# EUROPÄISCHE PATENTANMELDUNG

(11) **EP 3 693 739 A1**
(43) Veröffentlichungstag der Anmeldung: **12.08.2020**
(21) Anmeldenummer: 19155839.4
(22) Anmeldetag: 06.02.2019
(51) Int. Cl.: G01N 33/543, B03C 1/02

(54) **VERFAHREN UND VORRICHTUNG ZUR ISOLIERUNG VON GEWÜNSCHTEN ZELLEN AUS EINER PROBE NICHT-MAGNETISCHER BIOLOGISCHER MATERIALIEN**

(71) Anmelder: Schreier, Stefan, 93194 Walderbach (DE); SanoLiBio Co., Ltd, Samut Prakan 10540 (TH)
(72) Erfinder: SCHREIER, Stefan, 93194 Walderbach (DE); LANG, Ulrich, 11900 Penang (MY)
(74) Vertreter: Reitstötter Kinzebach

(57) **Zusammenfassung**

Die Erfindung betrifft ein Verfahren und eine Vorrichtung zur Isolierung von gewünschten Zellen aus einer Probe nicht-magnetischer biologischer Materialien, welche eine Suspension gewünschter Zellen und unerwünschter Zellen umfasst, umfassend die Schritte:
- Zugabe von magnetischen oder magnetisierbaren Partikeln zur der Probe unter Einwirkung eines starken Magnetfeldgradienten, wobei die Partikel Oberflächenanteile aufweisen, die mit Zielzellen eine spezifische Bindung eingehen können, wobei die Zielzellen entweder die gewünschten oder die unerwünschten Zellen umfassen;
- Inkubation der Probe unter Einwirkung eines mittleren Magnetfeldgradienten, der niedriger als der starke Magnetfeldgradient des Zugabeschritts ist;
- Waschen der mit den Partikeln beschichtete Zellfraktion mit einer Waschlösung zur Verringerung unspezifischer Bindung;
- Abscheiden der Zielzellen aus der Probe unter Einwirkung eines starken Magnetfeldgradienten, der höher als der mittlere Magnetfeldgradient des Inkubationsschritts ist.

## Beschreibung

Die vorliegende Erfindung betrifft ein Verfahren und eine Vorrichtung zur Isolierung von gewünschten Zellen aus einer Probe nicht-magnetischer biologischer Materialien.

### Technischer Hintergrund

Im Bereich der biologisch-medizinischen Forschung ist die Abscheidung von biologischen Materialien aus einer heterogenen Partikelsuspension für verschiedenste Analyseverfahren unumgänglich. Das besondere Interesse derartiger Abscheidungsverfahren gilt dabei der Anreicherung von Zellen und einzelligen Organismen, aber auch von Zellfragmenten, Bakterien, Einzellern, Viren, Proteinen, Peptiden oder Nukleinsäuren.

Wenn im Zusammenhang mit der vorliegenden Anmeldung der Einfachheit halber von "Zellen" gesprochen wird, so sollen unter diesem Begriff im breitesten Sinne Zellen mehrzelliger Organismen, einzellige Organismen, aber auch Zellfragmente und Viren, sowie einzelne Biomoleküle, wie Proteine, Peptide oder Nukleinsäuren verstanden werden.

Zur Verbesserten Abscheidung oder Herbeiführung der Abscheidbarkeit von schwer oder unzureichenden trennbaren biologischen Materialien stehen heute unter anderem Markierungsverfahren zur Verfügung, die häufig auf sogenannten Affinitätsreaktionen basieren. Weit verbreitete Abscheidungsverfahren basieren auf einer Markierung mit Fluoreszenzfarbstoffen oder auf einer magnetischen Abscheidung, bei der synthetische magnetische Partikel an abzuscheidende Zellen oder Zellfragmente beziehungsweise -bestandteile geheftet werden. Gegenüber einer fluoreszenzaktivierten Zellsortierung zeichnet sich die magnetische Abscheidung durch einen verminderten technologischen und finanziellen Aufwand aus.

Die magnetische Abscheidung erfolgt durch eine Markierung eines Zielmaterials mit Rezeptor- beziehungsweise Ligand-beschichteten magnetischen Partikeln. Dabei umfasst der Ausdruck "Zielmaterial" die Gesamtheit jeglicher Substanzen oder Strukturen, die mit dem biologischen Material assoziiert und zum Zustandekommen eines spezifischen Bindungspaares befähigt sind. Dabei beschreibt der Ausdruck "spezifische Bindungspaar" wiederum ein Paar von Substanzen oder eine Kombination oder einer Substanz und einer Struktur, die gegenseitig eine Neigung zur Interaktion zeigen und schließt Elemente wie Zellkomponenten, biospezifische Liganden und Rezeptoren mit ein. In diesem Sinne erfolgt die Markierung eines Zielmaterials durch Assoziation spezifischer Bindungspaare bestehend aus Ligand und Rezeptor. Der Ausdruck "Ligand" bezeichnet dabei die mit dem Zielmaterial verbunden, zur spezifischen Bindung befähigten Strukturen, wie beispielsweise Antigene und Haptene, die durch mindestens ein Epitop oder andere charakteristische Determinanten definiert sind. Der Begriff "Rezeptor" bezeichnet die Markierungssubstanz oder eine Gruppe von Markierungssubstanzen mit biospezifischer Bindungsaffinität zu einem bestimmten Liganden unter Ausschluss der Bindung mit anderen Substanzen. Unter den möglichen Markierungsrezeptoren finden sich beispielsweise moleklonale Antikörper und deren Fragmente, spezifische Bindungsproteine, wie beispielsweise ProteinA, Biotin, Streptavidin, Lecithin, Aptamere, Nukleinsäuren usw. Vorzugsweise sind derartige biospezifische Bindungen nichtkovalenter Natur, was eine schnelle Reaktion verspricht und unter Umständen reversibel ist.

Zum Stand der Technik für mögliche Präparationsverfahren von magnetischen Partikeln und deren Verwendung bei der Abscheidung von biologischen Materialien sei beispielhaft auf die Patente US4,884,088, US4,654,267, US4,452,773 und US5,597,531 verwiesen. Dabei besteht der Partikelkern aus magnetischen Materialien, wie z. B. Magnetit, bei dem es sich um ein ferromagnetisches Eisenoxid handelt. Eine Korngröße der Kristallteilchen von 30 Nanometer und mehr ist für die Ausprägung eines paramagnetischen Verhaltens erforderlich. Ein derartiges Material wird als magnetisch ansprechbar, magnetisierbar oder auch als superparamagnetisch bezeichnet, da es eine hohe magnetische Polarisierung nur unter Einwirkung eines äußeren magnetischen Feldes erzeugt. Diese Eigenschaft ist besonders attraktiv, um etwaige Partikelaggregationen nach der eigentlichen magnetischen Markierung durch einen eventuell verbleibenden Restmagnetismus ausschließen zu können.

Das magnetische Verhalten der Partikel ist ausschlaggebend für die sogenannte Anreicherungseffizienz. Die magnetischen Partikel besitzen eine maximale magnetische Suszeptibilität, die von der Anzahl und Korngröße der ferromagnetischen Kristalle innerhalb eines Partikels abhängig ist.

Im Allgemeinen lassen sich magnetische Abscheidungsverfahren in intrinsische und extrinsische Verfahren aufteilen. Magnetische Partikel mit Durchmessern unterhalb 100 nm bilden daher in Abwesenheit eines Magnetfeldes nur ein schwaches oder kein magnetisches Moment aus. Das Verhältnis derartiger Partikelgrößen ist allerdings das günstige Verhältnis von reaktiver Oberfläche zu Gesamtvolumen beziehungsweise Partikelmenge. Aus diesem Grund ist die magnetische Abscheidung unter Verwendung von sogenannten Nanopartikeln, die im Größenbereich von 30 nm bis 100 nm liegen, nur bei sogenannten Hochgradient-Magnetabscheidungsverfahren, wie sie beispielsweise in den Patenten US4,664,796, US5,200,084, und in den Patentanmeldungen WO96/26782A und EP0 942 766 A beschrieben werden.

Kommerzielle Systeme mit derartigen magnetischen Nanopartikeln mit einem Nominaldurchmesser von 50 nm werden von der Firma Miltenyi für die allgemeine Zellseparation mittels eines intrinsischen Abscheideverfahrens entwickelt. Hierfür bedient man sich einer magnetischen Trennkammer, die aus einer ferromagnetischen Matrix und einem nicht-magnetischen Behältnis besteht, welches in ein starkes magnetisches Feld eingebracht wird. Dabei werden starke magnetische Feldgradienten in der Größenordnung von bis zu 100 Tesla/cm innerhalb der magnetischen Trennkammer generiert. Nachteilig an derartigen Verfahren ist der hohe Separationsaufwand, der sich ungünstig auf die Prozessdauer, die Wirtschaftlichkeit und eine eventuelle Automatisierung auswirkt. Außerdem stellt die Gegenwart der großen Fremdoberfläche der magnetischen Trennkammermatrix ein Stressfaktor für lebende Organismen, wie beispielsweise Blutzellen dar. Außerdem wird eine erhöhte unspezifische Bindung beobachtet, also eine Bindung von Magnetpartikeln die nicht durch die Assoziation spezifischer Bindungspaare hervorgerufen wird.

Um die Nachteile einer magnetischen Trennkammer zu vermeiden und den technologisch erforderlichen Aufwand zu senken, jedoch gleichzeitig die Vorteile von Nanopartikeln zu nutzen, wurden extrinsische Hochgradient-Magnetabscheidungsverfahren entwickelt. Dabei kommen externe Magnetanordnungen, beispielsweise in einer Quadrupol- oder Hexapol-Konfiguration zum Einsatz, wie sie in dem Patent US5,186,827 beschrieben sind. Die dabei erzeugten Feldgradienten liegen bei 1,5 Tesla/cm und mehr, erfordern aber Partikeldurchmesser, die im Bereich von 150 nm bis 4 µm liegen. Insbesondere bei Verwendung von Partikeln mit Durchmessern von mehr als 1 µm können sehr einfache und günstige magnetische Abscheidungssysteme konstruiert werden, bei denen beispielsweise ein einfacher externer Dauermagnet am Probenbehälter angeordnet wird. Kommerzielle extrinsische Systeme werden beispielsweise von der Firma Dynal Inc., Great Neck, New York, unter der Produktbezeichnung Dynal MPC1 vertrieben.

Ein wesentliches Ziel bei der Weiterentwicklung magnetischer Abscheidungssysteme besteht in der Verbesserung der Anreicherungsqualität. Der Anreicherungsprozess besteht grundsätzlich aus einer Inkubationsphase zur magnetischen Markierung des biologischen Zellmaterials und der anschließenden magnetischen Abscheidung. Die Inkubationsphase verläuft üblicherweise in Ruhe, wenn magnetische Partikel mit einer Größe von unter 150 nm verwendet werden oder unter gelegentlicher Vermischung bei Verwendung größerer Partikel.

In dem deutschen Patent DE 10 2015 013 851 der vorliegenden Erfinder wird ein dynamisches magnetisches Beschichtungssystem beschrieben, bei dem die Inkubationsphase unter Einfluss eines Magnetfeldes bei Drehung des Inkubationscontainers durchgeführt wird. In diesem Verfahren konnte bereits eine erhöhte magnetische Beschichtung bei verkürzter Prozessdauer und somit eine Erhöhung der Effizienz beziehungsweise Reaktivität der Partikelsuspension erzielt werden. Der Begriff "dynamisches magnetisches Beschichten" beschreibt dabei einen aktiven Vorgang zur Beschichtung von biologischem Material mit magnetischen Partikeln. Genauer bezieht sich dieses Verfahren in bisherigen Ausführungen auf die erhöhte und beschleunigte Assoziation von magnetischen Partikeln aller Art mit Zelloberflächen. Man kann davon ausgehen, dass durch die Drehung eines Inkubationsbehälters in einem magnetischen Feldgradienten während der Inkubation des biologischen Materials mit magnetischen Partikeln ein Kollisionsverhalten hervorgerufen wird, das die Beschichtung begünstigt. Der Begriff "Kollisionsverhalten" soll die Bewegungsvorgänge zur Ausprägung aller Arten von Bindungen zwischen Reaktionspartnern gleichen und verschiedenen Typs umfassen. Zur Ausbildung von Bindungen ist das Kollisionsverhalten der Reaktionsteilnehmer entscheidend und kann durch die Zufallsbewegung, das lineare Moment eines magnetischen Partikels, die Frequenz oder Anzahl der Kollisionen pro Zeiteinheit zweier gleicher oder unterschiedlicher Reaktionspartner, die Summe der Kollisionen über einen Inkubationszeitraum eines Reaktionspartners mit unterschiedlichen Reaktionspartnern und die Dauer des Kontaktes zweier Reaktionspartner verstanden werden.

Von besonderem Interesse in der pharmazeutischen und biomedizinischen Forschung und Entwicklung ist die Isolation von sehr seltenen Zellen aus einer heterogenen Zellsuspension. So kann beispielsweise die Isolation, Quantifizierung und Charakterisierung bestimmter seltener Zellen oder Zellpopulationen aus einer Blutprobe dazu beitragen, unabhängig von anderen Testresultaten schnell und kostengünstig und in Echtzeit pathologische Befunde zu erstellen. Eine spezielle Anwendung ist dabei die Anreicherung von sogenannten zirkulierenden Tumorzellen (ZTZs), insbesondere der Nachweis des Vorkommens von zirkulierenden Tumorzellen in peripherem venösem Blut und im Frühstadium von Karzinom- und Sarkom-Erkrankungen. ZTZs haben daher das Potential, als unabhängiger, frühdiagnostischer Biomarker zu dienen, wodurch beispielsweise Entnahmen von Gewebeproben überflüssig werden können.

Ein Hauptproblem bei der Anwendung derartiger Biomarker in der klinischen Praxis stellt die effiziente Selektion von derartigen sehr seltenen Zellen dar. Prinzipiell stehen dabei Verfahren zur positiven Selektion oder Verfahren zur negativen Selektion zur Verfügung. Bei der positiven Selektion werden die gesuchten Zellen magnetisch markiert und von den nichtmarkierten unerwünschten Zellen getrennt, während bei der negativen Selektion die unerwünschten Zellen magnetisch markiert werden und von den nichtmarkierten, gewünschten Zellen getrennt werden. Die negative Selektion hat den Vorteil der Rezeptorunabhängigkeit, hat aber eine geringere Selektivität bei höherer Sensitivität im Vergleich mit der positiven Selektion.

Der Begriff Anreicherungsdilemma bezeichnet das Problem unzureichender Anreicherungseffizienz zwecks Anreicherung von seltenen Zellen. Genauer, können nur hohe Reinheiten unter hohem Verlust des Analyten erreicht werden. Umgekehrt lässt sich geringer Verlust des Analyten nur unter Erduldung geringer Reinheit der gewünschten Zellen bewerkstelligen. Der Begriff Anreicherungseffizienz wird in log Stufen angegeben und errechnet sich aus dem Verhältnis der Reinheiten vor Anreicherung bzw. danach. Die Reinheit soll sich auf die Population der kernhaltigen Zellen beziehen und schließt damit die roten Blutzellen und Thrombozyten aus. Es errechnet sich somit die Reinheit von seltenen Zellen mit zum Beispiel 10 Zellen pro Milliliter (ml) Blut vor Anreicherung als 10 Zellen in 6x106 Leukozyten und ergibt 0.00017%. Eine theoretische Anreicherungseffizienz von 5 log ergibt somit eine Reinheit von 16.7% nach der Anreicherung.

Magentische Abscheidungsverfahren zur Anreicherung von seltenen Zellen im Blut können durch Parameter wie zum Beispiel Probenaufbereitungsdauer, Verlust von gewünschten Zellen, Abreicherungseffizienz, Anreicherungseffizienz, Probenkosten oder Automatisierbarkeit charakterisiert werden. Im Stand der Technik werden Systeme beschreiben, welche die Prozessierung von Probenflüssigkeiten von mehreren Millilitern erlauben, so gut wie keine gewünschten Zellen verlieren und Zellverunreinigungen durch unerwünschte kernhaltige Zellen wie zum Beispiel Leukozyten nach Anreicherung den Bereich von 1000 bis 5000 Zellen nicht überschreiten. Ein derartiges System wurde zum Beispiel von Fachin et al. (Fachin, Fabio, et al. "Monolithic chip for highthroughput blood cell depletion to sort rare circulating tumor cells." Scientific reports 7.1 (2017): 10936) unter der Bezeichnung CTC-iCHiP vorgestellt. Dieses Verfahren stellt eine Lösung zur Anreicherung von seltenen Zellen durch die Trennung der roten und Weißen Blutzellen mittels dreier Chipmodule dar. Allerdings wurden Verluste von kleinen gewünschten Zellen bis zu 30% eingeräumt. Der Goldstandard in der Anreicherung mittels positiver Selektion von im Speziellen zirkulierenden Epithelzellen ist unter der Bezeichnung CellSearch bekannt (Allard, W. Jeffrey, et al. "Tumor cells circulate in the peripheral blood of all major carcinomas but not in healthy subjects or patients with nonmalignant diseases." Clinical cancer research 10.20 (2004): 6897-6904). Auch hier wurde hohe Zellrückgewinnung und relative geringe Mengen des Rückstandes an unerwünschten Zellen nach dem Anreicherungsprozesses erreicht. Weitere Fortschritt stellt das dynamische magnetische Beschichten dar, wobei die wesentliche Verbesserung in dem Aufwand an Partikeln und der Verfahrenszeit und weniger in der Anreicherungseffizienz lag (Schreier, Stefan, et al. "Advances in rare cell isolation: an optimization and evaluation study." Journal of Translational Medicine 15.1 (2017): 6). Diese Fortschritte können jedoch nicht als ausreichend bezeichnet werden. Im Allgemeinen sind die hohen Kosten für eine Probe kommerzieller Systeme nachteilig. Auf technischer Seite gibt es kein System ohne den sog. Bias. Die hohe Selektivität des Cellsearch-Systems für Epithelzellen hat eine geringe Sensitivität für die Anwendung in der Krebsdiagnostik bewiesen (Raimondi, Cristina, Chiara Nicolazzo, and Angela Gradilone. "Circulating tumor cells isolation: the "post-EpCAM era", Chinese Journal of Cancer Research 27.5 (2015): 461). Des Weiteren wurde festgestellt, dass die Expression des Oberflächenzellmembranmarkers CD326 nur im Falle von wenigen speziell kultivierten Krebszellen hoch ist. Die Validierung derartige Systeme bezieht sich somit auf die Verwendung künstlicher Zellsysteme. Im allgemeinen leiden bestehende Systemen zur Anreicherung von seltenen Zellen aus Blutproben oftmals unter dem Anreicherungsdilemma, d.h. unter zu hoher Leukozytenverunreinigung und stellt oftmals in Abhängig von der jeweiligen analytischen Anwendung eine Beeinträchtigung des analytischen Ergebnisses bzw. einen Mehraufwand dar. Es bedarf der fortgeführten Anreicherung bzw. Isolation durch Mikromanipulation. Im Besonderen wird für molekulare Analyseanwendungen in der sog. "Liquid Biopsy" der Krebsdiagnostik eine Mindestreinheit von 1 % gewünscht. Beim Testen von 10 ml Blut beträgt die wahrscheinliche Anzahl von gewünschten Zellen in Patienten mit Krebs-Frühstadium 10 zirkulierenden Tumorzellen (ZTZs) in ca. 6x10⁷ kernhaltige Blutzellen und insg. c.a. 6x10¹⁰ nicht kernhaltige Zellen (exl. Platlets). Für das Erreichen von 1 %-Reinheit müssen demnach max. 1x10³ unerwünschte kernhaltige Zellen übrigbleiben, was einer 4.77log Abreicherung unerwünschter kernhaltiger Zellen entspricht. Derartige hohe Anreicherungen höchst unterschiedlicher Zellen stellt immer noch eine technologische Herausforderung dar und ist ein Grund für vergleichsweise geringeren Erfolg in der Liquid Biopsie in Frühstadium-Krebs als in metastasierenden Krebspatienten. Fortschritt im Feld bedeutet daher eine weitere Reduzierung der Leukozytenkontamination um den Faktor 10 bei möglichst geringen Verlusten an gewünschten Zellen, schneller Prozessierung und geringeren Kosten. Als ein Ansatz zur Lösung des Anreicherungsdilemmas gilt die Verminderung des Verlustes von gewünschten biologischen Materialien in magnetischen Zellseparationssystemen. Verbesserungen bezogen sich hauptsächlich auf die Verminderung unspezifischer Bindung an Oberflächen wie zum Beispiel innerhalb von HGMS Trennkammern (DE68919715, US6020210, DE102007043281, Lin et al. 2013) oder der erhöhten Spezifizität von Reaktionspartnern (US662398, US7387897B2) oder nachfolgender Spezialbehandlung durch Rückgängigmachung von unspezifischer Bindung (WO2002071929A2).

Ein grundsätzliches Problem bei magnetischen Trennverfahren stellt die unspezifische Bindung von magnetischen Partikeln mit biologischem Material dar. Die unspezifische Bindung lässt sich im Detail auf Grund einer Vielzahl, größtenteils unbekannter Systemparameter meist nicht näher charakterisieren. Grundsätzlich kann man die unspezifische Bindung jedoch in eine reversible und eine nicht-reversible Bindung unterteilen. Bei der magnetischen Zellseparation von biologischen Materialen spielt beispielsweise die Oberfläche des biologischen Materials, wie beispielsweise eine Zellmembran, eine wichtige Rolle. Reversible unspezifische Bindung, die meist durch elektrostatische Anziehung zwischen Biomolekülen hervorgerufen wird, kann durch Waschschritte verringert werden. Im Gegensatz dazu spielen bei der irreversiblen unspezifischen Bindung vor allem Interaktionen zwischen Proteinen eine wesentliche Rolle. Reversible unspezifische Bindung kann beispielsweise durch eine Behandlung der Probe mit spezifischen Blockierungsreagenzien minimiert werden, die vor Beginn der eigentlichen magnetischen Separation oder während der magnetischen Inkubation und der magnetischen Abscheidung eingesetzt werden können. Irreversible unspezifische Verbindung kann auch durch Kreuzreaktivität von Rezeptoren mit verschiedenen Ligaden hervorgerufen werden, daher ist es wichtig, dass die verwendete Rezeptor-Liganden-Spezifizität möglichst genau bekannt ist. Des Weiteren wird der unspezifische Teil von Antikörpern, das sogenannte Fc-Fragment, von einer Vielzahl von Zelltypen, wie beispielsweise Monozyten, gebunden. Daher wird auch bei der direkten Beschichtung durch magnetische Partikel die vorherige Blockierung des Fc-Rezeptors oder die Verwendung von rekombinanten Antikörpervarianten, wie z. B. Fab oder F(ab)₂-Fragment empfohlen.

Die im Stand der Technik beschriebenen magnetischen Trennverfahren stellen keine zufrieden stellenden technologischen Ansätze für eine möglichst allumfassende Isolation von seltenen zirkulierenden Zellen in einem Konzentrationsbereich von einzelnen bis zu mehreren tausend Zellen in einer Probe dar, insbesondere bei Zellen beziehungsweise Zellfragmenten mit hoher Ligandenheterogenität und -plastizität, wie beispielsweise ZTZs.

### Technisches Problem

Durch das dynamische magnetische Beschichten, wie es in dem Patent
DE 10 2015 013 851 der vorliegenden Erfinder beschrieben ist, wird eine effiziente magnetische Beschichtung von biologischem Material erreicht. Allerdings geht mit dieser effizienten magnetischen Beschichtung auch eine erhöhte unspezifische Bindung des magnetischen Materials einher. Der vorliegenden Erfindung liegt daher das technische Problem zu Grunde, das aus DE 10 2015 013 851 bekannte Verfahren derart weiterzubilden, dass eine effizientere magnetische Trennung bei gleichzeitiger Reduzierung der Prozessdauer erreicht wird.

### Kurzbeschreibung der Erfindung

Gelöst wird dieses technische Problem durch das Verfahren des vorliegenden Anspruchs 1 und die Vorrichtung des vorliegenden Anspruchs 11. Vorteilhafte Weiterbildungen des erfindungsgemäßen Verfahrens und der erfindungsgemäßen Vorrichtung sind Gegenstand der abhängigen Patentansprüche.

Die Erfindung betrifft demnach ein Verfahren zur Isolierung von gewünschten Zellen aus einer Probe nicht-magnetischer biologischer Materialien, welche eine Suspension gewünschter Zellen und unerwünschter Zellen umfasst, wobei das Verfahren die Schritte umfasst:
- Zugabe von magnetischen oder magnetisierbaren Partikeln zu einer Probe unter Einwirkung eines starken Magnetfeldgradienten, wobei die Partikel Oberflächenanteile aufweisen, die mit Zielzellen eine spezifische Bindung eingehen können, wobei die Zielzellen entweder die gewünschten oder die unerwünschten Zellen umfassen;
- Inkubation der Probe unter Einwirkung eines mittleren Magnetfeldgradienten, der niedriger als der starke Magnetfeldgradient des Zugabeschritts ist;
- Waschen der mit den Partikeln beschichteten Zellfraktion mit einer Waschlösung zur Verringerung unspezifischer Bindung;
- Abscheiden der Zielzellen aus der Probe unter Einwirkung eines starken Magnetfeldgradienten, der höher als der mittlere Magnetfeldgradient des Inkubationsschrittes ist.

Das erfindungsgemäße Verfahren umfasst daher die herkömmlichen Schritte magnetischer Abscheideverfahren bestehend aus Zugabe der magnetischen Partikel, Inkubation, Waschen der Probe und Abscheiden der Zielzellen, wobei sich das erfindungsgemäße Verfahren jedoch dadurch auszeichnet, dass die einzelnen Schritte unter spezifisch unterschiedlichen Magnetfeldgradienten durchgeführt werden. Im Gegensatz zum Stand der Technik kann mit dem erfindungsgemäßen Verfahren die reversible unspezifische Bindung drastisch reduziert werden.

Wie bereits einleitend erläutert, ist im Rahmen des vorliegenden Verfahrens der Begriff "Zellen" weit auszulegen und umfasst insbesondere auch Zellfragmente und einzelne Zellbestandteile, wie zum Beispiel Organellen, pflanzliche, tierische Zellen und Pilzzellen und im weitesten Sinne biologisches Material. Im Speziellen schließt der Begriff "Zelle" zirkulierende Tumorzellen, Leukozyten, Keimbahn- und Stammzellen, Zellen des Knochenmarks in frühen und späten Entwicklungsphasen, Krebszellen, somatische Zellen, Zellen in Suspension, im Gewebe oder in einer Gewebsprobe und kernhaltige Zellen mit ein. Der Begriff seltene Zellen umfasst alle kernhaltigen als auch nicht-kernhaltige Zellen in sämtlichen Körperflüssigkeiten. Im Blut sollen seltene Zelle ausschließlich nicht das Antigen CD45 auch bekannt als "*common leukocyte antigert*" auf ihrer Zelloberfläche ausstellen. Darunter fallen alle nicht-hematopoetischen sowie hematopoetischen Zellen.

Mit dem Begriff "gewünschte Zellen" sind im Rahmen des vorliegenden Verfahrens die Zellen beziehungsweise Zellbestandteile gemeint, die vom Rest des biologischen Materials isoliert werden sollen. Der Begriff "unerwünschte Zellen" umfasst dementsprechend diejenigen Zellen und Zellbestandteile der Probensuspension, die nicht isoliert werden sollen und daher den Rest der Suspension darstellen.

Der Begriff "Zielzellen" beschreibt diejenigen Zellen, die mit den magnetischen oder magnetisierbaren Partikeln durch eine spezifische Bindung beladen werden sollen. Wenn es sich bei den Zielzellen um die gewünschten Zellen handelt, betrifft das erfindungsgemäße Verfahren ein Verfahren der positiven Selektion. Handelt es sich bei dem Zielzellen um die unerwünschten Zellen, betrifft das erfindungsgemäße Verfahren ein Verfahren der negativen Selektion.

Das erfindungsgemäße Verfahren kann mit magnetischen Partikeln durchgeführt werden, die bereits eine permanente Magnetisierung aufweisen oder mit magnetisierbaren Partikeln, die erst in einem externen magnetischen Feld eine Magnetisierung entwickeln.

Im folgenden werden die Begriffe "klein" und "groß" in Bezug auf magnetische Partikel wie folgt verwendet: Kleine Partikel bezeichnen Partikel mit Durchmessern bis zu 150 Nanometer (nm), größere Partikel haben Durchmesser von mehr als 150 nm. Im Zusammenhang mit Zellen oder Zellbestandteilen beziehen sich kleine Zellen auf Durchmesser unter 10 Mikrometer (µm), mittlere Zellen auf einen Bereich von 10 bis 13,5 µm und große Zellen auf Zellen mit Durchmessern von mehr als 13,5 µm.

Das erfindungsgemäße Verfahren wird bei zumindest zwei unterschiedlichen Stärken des Magnetfeldgradienten durchgeführt. Die Zugabe der magnetischen oder magnetisierbaren Partikel erfolgt unter einem starken Magnetfeldgradienten, während die anschließende Inkubation der Probe unter Einwirkung eines mittleren Magnetfeldgradienten erfolgt, der niedriger als der starke Magnetfeldgradient des Zugabeschritts ist. Beim Abscheiden der Zielzellen aus der Probe wird der Magnetfeldgradient gegenüber dem mittleren Magnetfeldgradienten des Inkubationsschritts wiederum erhöht.

Vorzugsweise lässt man die Probe während der Durchführung des erfindungsgemäßen Verfahrens bei unterschiedlichen Drehgeschwindigkeiten rotieren. So lässt man gemäß einer bevorzugten Ausführungsform der Erfindung die Probe während des Zugabeschritts der magnetischen oder magnetisierbaren Partikel mit einer schnellen Drehgeschwindigkeit rotieren.

Vorzugsweise besteht der Inkubationsschritt aus mehreren Inkubationszyklen, wobei jeder Inkubationszyklus
- einen magnetischen Beschichtungsschritt unter Einwirkung des mittleren Magnetfeldgradienten bei einer langsamen Drehgeschwindigkeit der Probe, die niedriger als die schnelle Drehgeschwindigkeit des Zugabeschritts, zur Erzeugung einer mit den Partikeln beschichteten Zellfraktion,
und
- einen Mischungsschritt zur Durchmischung der mit den Partikeln beschichteten Zellfraktion in der Suspension
umfasst.

Während des magnetischen Beschichtungsschritts wird die entstandene, mit Partikeln beschichtete Zellfraktion unter dem Einfluss des Magnetfeldgradienten zum Rand des Probengefäßes wandern und sich dort teilweise ablagern. Der anschließende Mischungsschritt in jedem Inkubationszyklus dient dazu, die Zellfraktion wieder in der Suspension zu verteilen, um die spezifische Beladung der Zielzellen mit Partikeln zu erhöhen und die unspezifische Beladung von Zellen zu verringern. Diese Durchmischung kann durch unterschiedliche Maßnahmen bewirkt werden, beispielsweise durch mehrfaches Absaugen und Wiedereinspritzen des Flüssigkeitsüberstandes, durch sog. Vortexen der Suspension oder ähnliche Maßnahmen. Der Magnetfeldgradient wird dabei üblicherweise niedriger als beim Beschichtungsschritt sein.

Das Durchmischen kann auch ohne Einwirkung eines Magnetfeldgradienten durchgeführt werden, besonders bevorzugt wird der Mischungsschritt jedoch unter Einwirkung eines geringen Magnetfeldgradienten, der niedriger als der mittlere Magnetfeldgradient des Beschichtungsschritts ist durchgeführt. Besonders bevorzugt erfolgt der Mischungsschritt bei einer schnellen Drehgeschwindigkeit der Probe, die höher als die langsame Drehgeschwindigkeit des Beschichtungsschrittes ist, um so eine besonders effektive Durchmischung zu gewährleisten.

Typischerweise umfasst der Inkubationsschritt drei bis zehn Inkubationszyklen.

Gemäß einer bevorzugten Ausführungsform des erfindungsgemäßen Verfahrens besteht der Waschschritt aus einem oder mehreren Waschzyklen und jeder Waschzyklus umfasst dabei die Schritte:
- Entfernen eines Überstandes der Suspension;
- Resuspendieren der verbleibenden, mit Partikeln beschichteten Zellfraktion in der Waschlösung bei einem geringen Magnetfeldgradienten, der niedriger als der mittlere Magnetfeldgradient des Beschichtungsschrittes ist bei einer schnellen Drehgeschwindigkeit, die höher als die langsame Drehgeschwindigkeit des Beschichtungsschrittes ist;
- Fraktionieren der mit den Partikeln beschichteten Zellfraktion aus der Suspension bei einem starken Magnetfeldgradienten, der höher als der mittlere Magnetfeldgradient des Beschichtungsschrittes ist bei einer mittleren Drehgeschwindigkeit, die niedriger als die schnelle Drehgeschwindigkeit des Resuspendierschrittes ist.

Der in jedem Waschzyklus entfernte Überstand wird gesammelt und kann abhängig davon, ob das erfindungsgemäße Verfahren auf positive oder negative Selektion ausgerichtet ist, unterschiedlich verarbeitet werden. Dabei bezieht sich die unterschiedliche Verarbeitung zum einen auf die Intensität der Abscheidung (beispielsweise auf Dauer und die Stärke des Magnetfeldgradienten) und zum anderen auf den Zeitpunkt, bei dem der Überstand verworfen wird. In der negativen Selektion befinden sich die gewünschten Zellen komplett im Überstand, so dass eine sehr intensive magnetische Abscheidung erforderlich wird, um die Zielzellen, die demnach die unerwünschten Zellen darstellen, zu entfernen. Der Überstand bildet damit die neue Probe für weitere Prozesse. Bei der positiven Selektion befinden sich die Zellen in der ersten magnetischen Fraktion, die während des Waschprozess am Gefäßrand abgeschieden wird und in der zweiten magnetischen Fraktion, die nach der magnetischen Abscheidung entsteht. Die Abscheidung muss also nicht so stark sein, wie bei der negativen Selektion und der Überstand wird nach der magnetischen Abscheidung verworfen oder für weiterführende Zellseparationsprozesse verwendet. Der Überstand muss also lokal von der zweiten magnetischen Fraktion getrennt werden, da die zweite magnetische Fraktion, wie auch die erste magnetische Fraktion, in einer neuen, sauberen Pufferlösung resuspendiert werden müssen. Die resuspendierten und auf geeignete Weise zusammengeführten magnetischen Fraktionen bilden dann die neue Probe.

Bevorzugt führt man den Waschzyklus ein bis fünf Mal durch.

Der Abscheideschritt dient dazu, die Zielzellen magnetisch zu fixieren und die restliche Suspension von den Zielzellen zu trennen. Vorzugsweise lässt man die Probe bei dem Abscheideschritt nicht rotieren. Gemäß einer bevorzugten Ausführungsform ist der starke Magnetfeldgradient des Abscheideschritts sogar höher als der starke Magnetfeldgradient des Zugabeschritts, damit eine möglichst eine effektive Fixierung der Zielzellen erreicht und somit möglichst wenigstens wenige Zielzellen mit der Suspension entfernt werden.

Gemäß der Variante des erfindungsgemäßen Verfahrens zur positiven Selektion enthalten die Zielzellen die gewünschten Zellen. Bei der Verfahrensvariante zur negativen Zellselektion sind die gewünschten Zellen in der entfernten Suspension enthalten.

Die im erfindungsgemäßen Verfahren verwendeten Partikel enthalten vorzugsweise magnetisierbare superparamagnetische Materialien. Bevorzugt haben die magnetischen oder magnetisierbaren Partikel eine Größe (Durchmesser oder hydrodynamischer Durchmesser) der im Bereich von 100 nm bis 4 µm und besonders bevorzugt im Bereich von 750 nm bis 1 µm liegt.

Gemäß einer Variante des erfindungsgemäßen Verfahrens inkubiert man die Probe vor der Zugabe der magnetischen oder magnetisierbaren Partikel in einer Pufferlösung, die Makromoleküle zur Sättigung unspezifischer Bindungsstellen enthält.

Der Begriff "unspezifische Bindung" (USB) soll im Folgenden für die temporäre oder dauerhafte Assoziation von biologischen Materialien mit sämtlichen im Anreicherungsprozess auftretenden Oberflächen stehen, die nicht als erwünscht deklariert sind. Der Begriff irreversible bzw. reversible USB soll sich auf die Bindung zwischen magnetischen Partikeln und Zelloberfläche beziehen. Irreversible Bindung soll bedeuten, dass die Bindungspartner nur unter zusätzlicher Anwendung spezieller Dissoziationsverfahren dissoziiert werden können. Irreversible USB geht daher bei negativer Selektion mit dem Verlust von erwünschten Zellen durch unerwünschte magnetische Beschichtung und die daraus folgende magnetische Abscheidung einher. Der Begriff reversible USB soll bedeuten, dass unter Verwendung zellschonender Waschverfahren eine Dissoziation magnetische Partikeln von Zelloberflächen möglich ist. Mit dem erfindungsgemäßen Verfahren kann neben einer Verringerung der reversiblen unspezifischen Bindung auch die irreversible unspezifische Bindung verringert werden.

Für eine derartige Pufferlösung wird im Folgenden auch der Begriff "Blockierlösung" verwendet. Dieser Begriff bezeichnet also eine Substanz zur angestrebten Verhinderung von unspezifischer Bindung der Zellen mit allen während des Anreicherungsprozess vorkommenden Oberflächen benennen. Derartige Substanzen schließen mit ein, sind jedoch nicht begrenzt auf: globuläre Proteine wie Albumin, Kuh-, Kälber- und Human- Serumalbumin, Ovoalbumin, Lactoalbumin oder pflanzliches Albumin, Beta-Lactoglobulin, Kappa-Kasein, Histone, Protamine, Globuline, Prolamine, Glutenline oder filamentartige Proteine, wie zum Beispiel Gelatinen aller Art, im Besonderen vom Fisch und Schwein, vorzugsweise in einer Konzentration von 0.1 % bis 5 % Gew.%. Im Besonderen eignen sich Kuhserumalbumin von 3 % bis 5 % und Fischgelatine von 2% bis 3% Gew.% zur effizienten Blockierung von unspezifischer Bindung.

Das erfindungsgemäße Verfahren kann zur Anreicherung der gewünschten Zellen mehrfach durchgeführt werden. Zwischen den einzelnen Verfahrensdurchläufen ist dann, insbesondere bei einem auf negative Selektion ausgerichteten Verfahren, vorzugsweise ein Zentrifugierschritt vorgesehen, um die gewünschten Zellen aus einer nach Abschluss eines Verfahrens durch Laufs abgelassenen Suspension abzutrennen. Die gewünschten Zellen befinden sich nach einem Verfahrensdurchlauf in einem ackumulierten großen Suspensionsvolumen. Mit dem Zentrifugieren wird wieder entsprechend geringes Inkubationsvolumen für den folgenden Durchlauf des Verfahrens bereitgestellt, um die erforderliche hohe Partikelkonzentration bzw. Zielzellkonzentration und mindesterforderliche Partikelmenge zu erreichen. Die Wiederholung mit Zentrifugation als Zwischenschritt bietet sich auch für die positive Selektion an, falls die Zielzellenanreicherung einen sehr hohen Anteil erreichen soll.

Die Erfindung betrifft außerdem eine Vorrichtung zur Beladung von biologischem Material mit magnetischen oder magnetisierbaren Partikeln, umfassend wenigstens eine drehbare Halterung zur Aufnahme eines Probenbehälters, die durch einen mit der Halterung gekoppelten Antriebsmotor in eine Drehung mit veränderbarer Drehgeschwindigkeit versetzt werden kann;
eine Magnetisierungseinrichtung, die am Ort des Probenbehälters ein Magnetfeld mit veränderbarer Feldstärke und veränderbarem Feldgradienten erzeugen kann, und
eine Steuerungseinrichtung zur Steuerung des Antriebsmotors und der Magnetisierungseinrichtung.

Die Magnetisierungseinrichtung kann in unterschiedlicher, dem Fachmann bekannter Weite realisiert werden. Beispielsweise können ein oder mehrere Elektromagneten verwendet werden, deren Ausrichtung und Stromstärke das veränderbare Magnetfeld und den veränderbaren Magnetfeldgradienten erzeugen. Eine besonders einfache und kostengünstige Vorrichtung lässt sich jedoch unter Verwendung von einem oder mehreren Permanentmagneten realisieren, beispielsweise aus hartmagnetischen Werkstoffen, wie zum Beispiel Neodym, Eisen oder Hartferrite aus Eisenoxid, sowie Barium- bzw. Strontiumcarbonat, PtCo-Legierungen und viele Weiteren.

Gemäß einer bevorzugten Ausführungsform der Erfindung weist der Probenbehälter eine im Wesentlichen zylindrische Geometrie mit einer Zylinderachse auf und die Magnetisierungseinrichtung umfasst einen Permanentmagneten, der ein Magnetfeld erzeugt, dessen Feldstärke parallel zu einer Längsachse des Magneten konstant ist und in Ebenen senkrecht zu dieser Längsachse einen Feldgradienten ausbildet, wobei der Abstand der Längsachse des Magneten zur Zylinderachse des in der Halterung befindlichen Probenbehälters veränderbar ist. mit dieser Ausführungsform der erfindungsgemäßen Vorrichtung können über die Höhe des Probenbehälters gleichförmige magnetische Bedingungen erzielt werden, während in Ebenen senkrecht zur Längsachse des Probenbehälters das Magnetfeld ausgebildet wird, welches Kräfte auf die magnetischen oder magnetisierbaren Partikel ausübt, die zur Beladung und Abtrennung der Zielzellen erforderlich sind.

Besonders bevorzugt ist außerdem die Neigung der Längsachse des Magneten bezüglich der Zylinderachse des in der Halterung befindlichen Probenbehälters veränderbar, so dass insbesondere beim Waschschritt und/oder beim Abscheideschritt eine an die spezifische Form des Probenbehälters angepasste Geometrie gewählt werden kann. Wird beispielsweise ein Probenbehälter mit einer im Wesentlichen zylindrischen Geometrie verwendet, dessen Bodenbereich konisch ausgebildet ist (typische im Laborgebrauch üblichen Behälter und -küvetten weisen beispielsweise eine um ca. 11° nach konisch nach Innen geneigte Behälterwand im unteren Gefäßbereich auf), so kann durch Neigen des Magneten entsprechend dem Konuswinkel gerade in der Phase des Waschschrittes eine größtmögliche magnetische Wirkung erzielt werden. Generell verbessert diese geometrische Anpassung die Homogenität des Magnetfeldgradienten im Behälter in vertikaler Achse und wirkt sich so auch in anderen Schritten des Anreicherungsverfahrens positiv aus.

Gemäß einer bevorzugten Ausführungsform umfassend die erfindungsgemäße Vorrichtung außerdem Mittel zur Isolierung von gewünschten Zellen aus der Probe biologischer Materialien, wobei die Mittel zur Isolierung eine beweglich montierte, von der Steuerungseinrichtung gesteuerte Spritze umfassen, die einerseits zum Austausch von Fluiden mit dem Probenbehälter in Wirkung gebracht werden kann und andererseits zum Ablassen von Fluiden mit der Magnetisierungseinrichtung in Wirkverbindung gebracht werden kann. Darüber hinaus kann die Spritze auch zur Unterstützung der Durchmischung verwendet werden, indem beispielsweise Suspension in die Spritze gesaugt und wieder in den Probenbehälter zurückgespritzt wird.

Besonders bevorzugt ist die Spritze neigbar montiert, so dass der Abscheideprozess kontrollierter gestaltet werden kann, weil je nach Neigung der Spritze das Ablassen des Fluidüberstandes mehr oder weniger schnell erfolgt.

Vorzugsweise sind der Probenbehälter und die Spritze zur Aufnahme von Fluidvolumina im Bereich von 10 µl bis 100 ml ausgelegt.

In einer Ausführungsform umfasst die erfindungsgemäße Vorrichtung eine Inkubationsapparatur und eine Abscheidungsapparatur, die im Wesentlichen aus einer Pipettierapparatur mit einem zusätzlichen Freiheitsgrad, dem der Neigung in vertikaler Ebene, besteht und sich der Magneten der Inkubationsapparatur bedient. Der Begriff Inkubationsapparatur bezieht sich im Speziellen auf die Funktionalität der spezifischen Beladung von magnetischen Partikeln an biologischem Material. Der Begriff Pipettierapparatur beschreibt eine fahrbare Achse in drei Freiheitsgraden, die eine Plastikspritze zum Befüllen und Ablassen von Flüssigkeiten trägt. Der Begriff Abscheidungsapparatur bezieht sich im Speziellen auf die Funktionalität des magnetischen Abscheidens von im vorherigen Verfahrensschritt magnetisiertem biologischem Material.

In einer bevorzugten Ausführungsform können die Stärke des Magnetfeldgradienten und die Drehgeschwindigkeit in den einzelnen Verfahrensschritten in der folgenden Tabelle 1 zusammengefasst werden:

**Tabelle 1**

| Verfahrensschritt | Magnetfeldgradient | Drehgeschwindigkeit | Dauer |
|---|---|---|---|
| Partikelzugabe | stark | schnell | kurz |
| Inkubation/Beschichtung | mittel | langsam | lang |
| Inkubation/Mischung | gering | schnell | mittel |
| Waschung/Mischung | gering | schnell | mittel |
| Waschung/Fraktionierung | stark | mittel | mittel |
| Abscheidung | stark | keine Drehung | sehr lang |

In Tabelle 1 beziehen sich die relativen Angaben des Magnetfeldgradient typischerweise auf einen Bereich von 0.5 bis 100 Tesla pro Zentimeter (T/cm), die Drehgeschwindigkeit auf einen Bereich von 0 bis 1000 Umdrehungen pro Minute (min⁻¹) und die Dauer auf einen Bereich von 0 bis 600 Sekunden (s).

Die Stärke des Magnetfeldgradienten kann bei Verwendung eines Permanentmagneten beispielsweise in erster Linie durch den Abstand zwischen Probenbehälter und Magnet beeinflusst werden. Je geringer der Abstand desto höher ist der Gradient. Ein hoher Gradient erhöht die kinetische Energie der Partikel.

Die Drehgeschwindigkeit spielt sowohl für die Fraktionierung der magnetisch beladenen Zellen, als auch die Kontaktdauer zwischen magnetischen Partikeln und biologischem Material eine Rolle. Je schneller gedreht wird, desto geringer die Fraktionierung und desto geringer die Kontaktdauer. Je nach Verfahrensschritt wird einer der beiden Aspekte der Drehung wichtig.

Zusammenfassend soll herausgestellt werden, dass die Erfindung eine Reihe von Verbesserung mit sich bringt. Die Erfindung ist im Besonderen durch die hohe Effizienz für die Abreicherung stark überzähliger Mengen an Zielzellen geeignet. Die Effizienz bezieht sich zum einen auf die Schnelligkeit und den hohen Reaktivitätsgrad der Partikel. Da die Partikel den wesentlichsten Teil zu den Kosten in derartigen Verfahren beitragen, stellt die Erfindung eine wesentliche Kostenerleichterung bei der Durchführung eines magnetophoretischen Zellisolierverfahrens dar. Generell liegt die Dauer des Anreicherungsprozesses ab Vermischung der Zellsuspension mit den magnetischen Partikeln bis hin zum Erhalt der aufgereinigten Lösung im Bereich von wenigen Minuten, was als neue Klasse von Schnelligkeit bei derartigen Verfahren zu betrachten ist. Generell hat dies den Vorteil sehr hoher Probendurchsätze, was sich beflügelnd im Forschungsbetrieb als auch für den Einsatz in der klinischen Routine auswirken kann. Im Besonderen erlaubt die kurze Dauer auch eine Wiederholbarkeit des Verfahrens innerhalb biologisch vertretbarer Prozessdauer, was für die Hochanreicherung in der negativen Selektion entscheidend ist. Des Weiteren ist die Einstellbarkeit der Beschichtungseffizienz von Vorteil und wird durch die erfundene Apparatur unterstützt. Das Verfahren erlaubt hohe Reproduzierbarkeit. Kontrollierte Beschichtung sowohl niedrig als auch hoch, erlaubt hohe Abreicherung bei der Variante der negativen Selektion und sanfte Beschichtung bei der positiven Selektion. Wie unten detaillierter ausgeführt wird, kann dies durch die Einstellung der Bead-Menge und der Art und Anzahl der Inkubationszyklen bewerkstelligt werden. Die wesentliche Verbesserung ist die Problemlösung des Anreicherungsdilemmas, was sich förderlich auf die Anreicherung von seltenen Zellen mittels negativer Selektion auswirkt. Dies wurde in der Erfindung durch die Erhöhung der Partikelbindungseffizienz, der Erniedrigung der magnetischen Ansprechbarkeitsschwelle in der Abreicherungsapparatur und durch die Erkenntnis der Reduzierung der USB ermöglicht. Es soll mit dieser Technik nicht wie bisher im 2-3log Raum sondern im 4 -6log Stufen gearbeitet werden und somit auch bei negativer Selektion die Reinheitsgrade der positive Selektion zu erreicht werden. Weiter ist zu erwähnen ist der Vorteil des Arbeitens mit relativ kleinen Volumina, so dass hier auch Automatisierbarkeit erleichtert bzw. ermöglicht wird. Prinzipiell ist das Verfahren für positive als auch negative Anreicherung anwendbar.

Die Erfindung kann besondere Anwendung in der Anreicherung von seltenen Zellen mittels negativer Selektion finden. Es versteht sich, dass nur sehr sensitive Methoden Zugang zu diesen Zellarten ermöglichen. Anwendungsbereich betritt die sog. Liquid Biopsy, mit der versucht wird im Speziellen zirkulierende Tumorzellen zu Detektieren, Isolieren und Kultivieren. Das Prinzip der negativen Selektion basierend auf der Erfindung erlaubt nicht-selektive Anreicherung und begünstigt daher diagnostische Verfahren mit hoher Sensitivität und Kultivierbarkeit von zirkulierenden Tumorzellen. Durch den geringen Verlust von gewünschten Zellen lassen auch unbekannte Zellarten identifizieren, die für zukünftige Weiterentwicklung der diagnostischen Liquid Biopsy und pharmakologischen Produktentwicklung von Interesse wäre. Ein dabei sicherlich neues Feld ist die Analyse eines seltenen Zellspektrums des Blutes. Des Weiteren bedient die Erfindung das Feld der nicht-invasiven pränatalen Diagnostik, bei der fötale Zellen im Speziellen fötale Erythroblasten zur Genanalyse isoliert werden können. Des Weiteren wurde Forschung zur Vorhersage von Herzinfarktrisiken mittels der Analyse von seltenen Zellen im besonderen Masse zirkulierende Endothelzellen gemacht.

### Detaillierte Beschreibung der Erfindung

Die Erfindung wird im Folgenden anhand einer in den beigefügten Zeichnungen dargestellten bevorzugten Ausführungsform detaillierter beschrieben. In den Zeichnungen zeigen:
- Fig. 1: eine Seitenansicht des unteren Bereichs einer erfindungsgemäßen Vorrichtung zum Beladen von biologischem Material mit magnetischen oder magnetisierbaren Partikeln;
- Fig. 2: eine Draufsicht auf die Vorrichtung der Fig. 1;
- Fig. 3: eine Seitenansicht der Vorrichtung der Fig. 1 in einem anderen Prozessstadium, wobei der Magnet geneigt ist;
- Fig. 4: eine Ansicht des oberen Bereichs der Vorrichtung der Fig. 1, wobei der Magnet an die Spritze angelegt ist;
- Fig. 5: eine schematische Querschnittsdarstellung der in der Fig. 4 verwendeten Spritze;
- Fig. 6: den Grad der Abscheidung und des Verlustes der Leukozyten aus der Suspension in Abhängigkeit der Inkubationsmethode gemäß Beispiel 2;
- Fig. 7: die abscheidbare Zellmenge pro µg Ab als Funktion der Zielzellkonzentration in der Suspension aus Beispiel 4;
- Fig. 8: große, sich teilende Zelle bei einem Magenkrebspatienten aus Beispiel 7; und
- Fig. 9:: zirkulierende Epithelzelle bei einem Krebspatienten aus Beispiel 7.

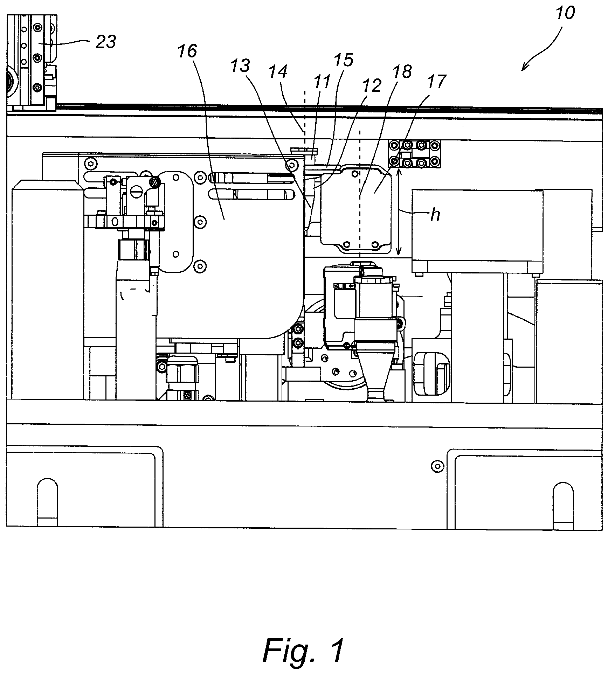

In Fig. 1 erkennt man den unteren Bereich einer insgesamt mit der Bezugsziffer 10 bezeichnete Vorrichtung zur Beladung von biologischem Material mit magnetischen oder magnetisierbaren Partikeln, wobei insbesondere der untere Teil der Vorrichtung erkennbar ist. Man erkennt einen im Wesentlichen zylindrischen Probenbehälter 11, der einen zylindrischen Mantel 12 und einen konischen Boden 13 aufweist. Eine Längsachse des Probenbehälters 11 ist durch das Bezugszeichen 14 symbolisiert. Der Probenbehälter 11 ist in einer drehbaren Halterung 15 angeordnet, die mit einer Antriebseinrichtung 16 gekoppelt ist. Unmittelbar neben dem Probenbehälter 11 erkennt man einen Permanentmagnet 17, dessen Längsachse durch das Bezugszeichen 18 symbolisiert wird.

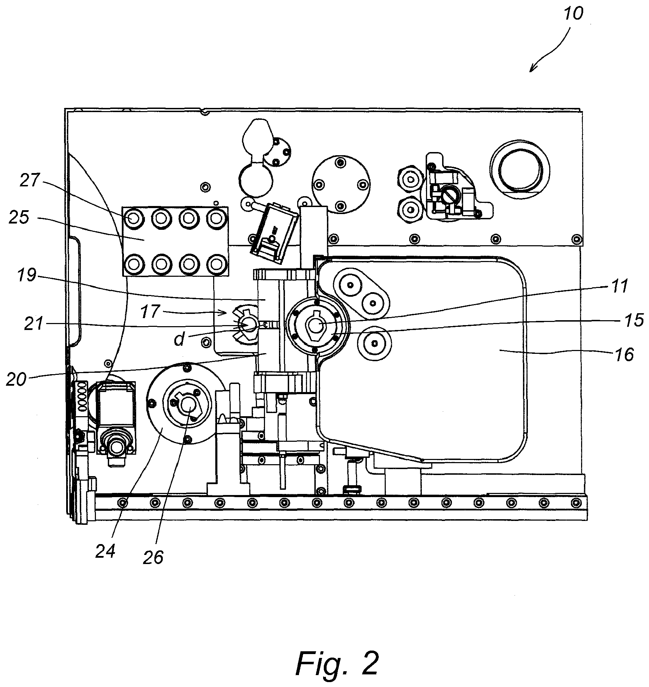

Wie insbesondere aus der Darstellung der Fig. 2 deutlich wird, weist der Permanentmagnet 17 zwei Pole 19, 20 auf, die durch einen Spalt 21 mit einer Spaltbreite d voneinander getrennt sind. Die Stärke des Permanentmagneten und die Spaltbreite d beeinflussen den im Außenbereich des Spaltes erzeugten Feldgradienten. In dieser Konfiguration ist das Magnetfeld über die Höhe des Permanentmagneten 17, also parallel zu dessen Längsachse 18 im Wesentlichen konstant. Außerhalb des Spaltes 21 entsteht in radialer Richtung, also in Ebenen senkrecht zu der Längsachse 18 des Permanentmagneten 17 ein inhomogenes Magnetfeld. Am Ort des Probenbehälters 11 ist das Magnetfeld in der in den Figuren 1 und 2 dargestellten Anordnung über die Höhe des Probenbehälters also im Wesentlichen konstant, in Ebenen senkrecht zu der Längsachse 14 des Probenbehälters übt der Magnetfeldgradient des inhomogenen Magnetfeldes jedoch eine Kraft auf magnetische oder magnetisierbare Partikel in dem Probenbehälter 11 auf.

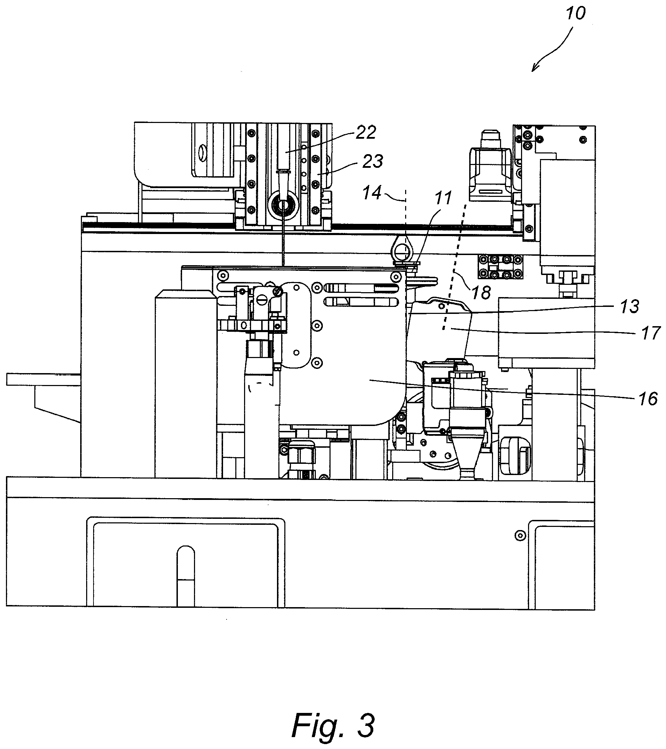

Durch Variation des Abstandes zwischen Probenbehälter 11 und Permanentmagnet 17, sowie durch Veränderung der Neigung der Längsachsen 14, 18 von Probenbehälter und Magnet zueinander können unterschiedliche Magnetfeldstärken und Magnetfeldgradienten erzeugt werden in dem Probenbehälter 11 erzeugt werden. Wie insbesondere aus der Darstellung der Fig. 3 hervorgeht, lassen sich sowohl die Position des Permanentmagnet 17 als auch dessen Neigung bezüglich dem Probenbehälter 11 verändern. In der Fig. 3 befindet sich der Permanentmagnet 17beispielsweise näher am Probenbehälter 11 und ist außerdem bezüglich der Längsachse 14 des Probenbehälters geneigt.

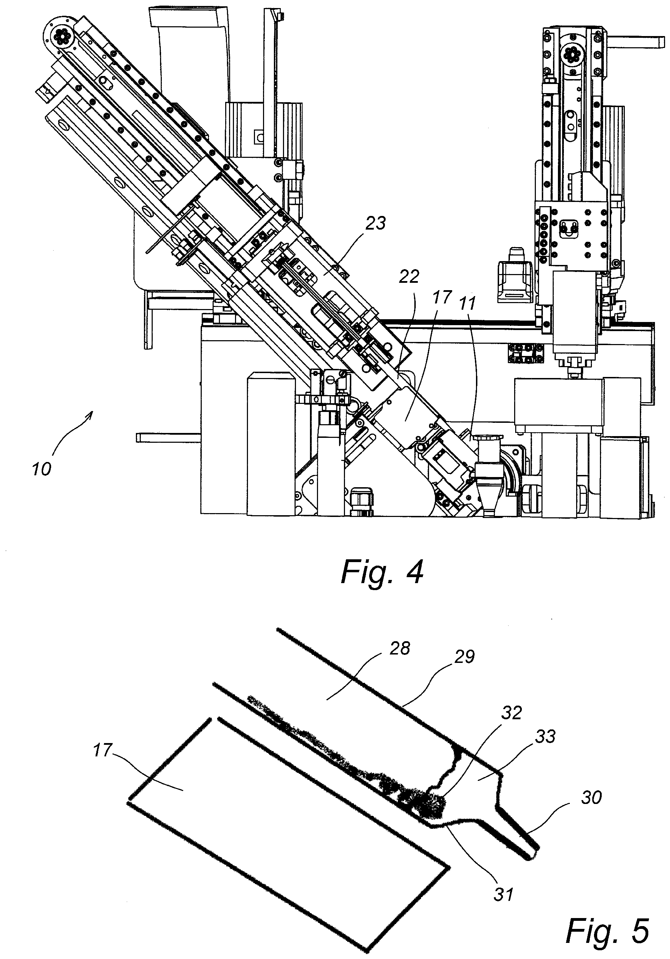

In der Darstellung der Fig. 4 ist der obere Bereich der erfindungsgemäßen Vorrichtung 10 dargestellt, so dass man insbesondere eine Spritze 22 erkennt, die sich in einer sowohl in der Neigung als auch in der Position bewegbaren Halterung 23 befindet, so dass beispielsweise Fluid aus dem Probenbehälter 11 in die Spritze 22 gefördert werden kann und umgekehrt. Außerdem erkennt man insbesondere in den Fig. 1 und 2 Halterungen 24, 25 mit weiteren Probenbehältern 26 beziehungsweise Fluidgefäßen 27, die ebenfalls von der Spritze angesteuert werden können.

In Fig. 5 ist eine bevorzugte Ausführungsform der Spritze 22 dargestellt, die einen Hauptkörper 28 mit einem im Allgemeinen zylindrischen Mantel 29 aufweist, der in einem Auslass 30 mit geringerem Durchmesser endet. In einem bauchigen Übergangsbereich 31 zwischen Hauptkörper 28 und Auslass 30 kann sich bevorzugt die in dem magnetischen Abscheideprozess zurückgehaltene Fraktion 32 mit magnetisierten Zielzellen ablagern, während der Überstand 33 durch den Auslass 30 abfließen kann.

Die erfindungsgemäße Vorrichtung ist prozessorgesteuert und so programmierbar, dass das erfindungsgemäße Verfahren in einer oder in mehreren Wiederholungen praktisch automatisch ablaufen kann.

### Weitere Ausgestaltungen der Erfindung

Aus Beobachtungen in Experimenten zur magnetischen Beschichtung von biologischen Material konnten tiefere Einblicke und ein tieferes Verständnis für den Beschichtungsvorgang gewonnen werden und einen Zusammenhang zwischen Reaktionskinetiken von spezifischer und unspezifischer Bindung während der Inkubation der magnetischen Partikel mit der Zellsuspension erkannt werden. Genauer konnte beobachtet werden, dass spezifische zu Gunsten unspezifischer Bindung bei geeigneter Apparatur und Protokollierung des Vorgangs erreicht durch vorteilhafte Einflussnahme auf die Bewegung der magnetischen Partikel erreicht werden kann. Für die Ausprägung von spezifischer und unspezifischer Bindung wird generell ein Bindungsvorteil für spezifische Bindung angenommen, woraus sich ein genereller Aufreinigungseffekt ableiten lässt. Es ist bekannt, dass die Affinität spezifischer Bindung der Antikörper-Antigen Bindung deutlich höher liegt als die der unspezifischen Bindung. Oft wird Antikörperbindungsfähigkeit als Inverses zur Affinität der Dissoziationskonstante angeben, welche im Fall von Antikörpern zwischen 10⁻⁷ und 10⁻⁹ und in selteneren Fällen bis zu 10⁻¹² betragen kann. Im Allgemeinen überschreiten die Dissoziationskonstanten unspezifischer Bindung nicht den mikromolaren Bereich (10⁻⁴-10⁻⁶) und das Bindungsverhalten ist oft linear (Peter Hein Kapitel 6). Die unspezifische Bindung darf im Gegensatz zur Antigen Antikörper Bindung nicht als schnelle Schlüssel-Schloss-Bindung mit einer definierten Bindungskonstante, sondern als Bereich von Klebrigkeit bis hin zur Irreversibilität verstanden werden. Des Weiteren wird angenommen, dass die Anzahl möglicher Bindungsstellen für spezifische Bindungspartner verhältnismäßig überwiegend sind. Es ist anzunehmen, dass die Partikelkinetiken unter Einfluss von verschiedenen magnetischen Stärken der spezifischen als auch der unspezifischen Bindung förderlich sind.

Das Wissen und die Annahmen über Bindungsverhalten zwischen spezifischer und unspezifischer Bindung haben zu neuen Theorien zur Verhinderung der unspezifischen Bindung geführt. Es lassen sich durch das neue Verfahren eine Reihe von Eigenschaften beeinflussen die der Kollisionstheorie zu Grunde liegen. Bekanntermaßen sind Partikelkonzentration und Reaktivität bzw. Spezifizität maßgeblich für das Gelingen der magnetischen Beschichtung. Diese Zusammenhänge lassen sich durch physikalische Eigenschaften besser erklären. Es kann angenommen werden, dass die magnetischen Partikel in Lösung bei Raumtemperatur ohne den Einfluss eines magnetischen Feldes je nach Größe einer bestimmten Zufallsbewegung oder passiven Bewegung unterliegen. Die Bewegung von sehr kleinen Partikeln im Durchmesserbereich von 30 nm bis 250 nm wird dabei von der Brownschen Molekularbewegung beeinflusst. Bei größeren Partikeln bis zu 4µm dominiert die Gravitation und führt zu Sedimentationsbewegung. Weiter wird angenommen, dass die hohe Zufallsbewegung sehr kleiner Partikel eine im Vergleich zu größeren Partikeln höhere Anzahl an Kollisionen pro Zeiteinheit in einer Volumenausbreitung weniger vielfacher Dimensionen des Partikeldurchmessers (lokale Kollisionsfrequenz) erzeugt und sich damit in Gegenwart eines Liganden bindungsfördernd gemessen über einen bestimmten Zeitraum auswirkt. Eine hohe Kollisionsfrequenz mag von Vorteil für das Zustandekommen einer lokalen Bindungsmöglichkeit unter der Annahme der höheren Wahrscheinlichkeit auf Ausbildung spezifischer gegenüber der unspezifischen Bindung sein. Diese passive Partikelbewegung während des magnetischen Beschichtungsvorgangs kann durch ein entsprechendes magnetisches Feld beeinflusst werden. Das heißt bei sehr kleinen Partikeln wird die Zufallsbewegung minimiert und bei größeren Partikeln wird die Schwerkraft überlagert. Es soll hier von einer magnetischen Zugkraft auf die Partikel hin zum höchsten magnetischen Gradient gesprochen werden, die durch die magnetischen Suszeptibilität des Partikels einerseits und andererseits durch die Höhe des einflussnehmenden Feldgradienten bestimmt ist. Diese magnetische Zugkraft führt zu einer in eine gerichteten Geschwindigkeit in Abhängigkeit der Viskosität des Mediums und etwaigen nichtmagnetisierbaren Hindernissen im Medium. Somit nehmen die Partikel bestimmte lineare Momente mit dem Potential zur Kollision mit immobilen Liganden innerhalb eines Volumens über den gesamten Behälterquerschnitt in Abhängigkeit des Zeitintervalls an. Es wird des weiteren angenommen, dass die Kollision zwischen Zelle und Partikel mit einem bestimmten Grad an Klebrigkeit verbunden ist und somit in Auftreff- und Ablösungszeitpunkt unterschieden werden kann, so fern sich keine permanente Bindung ergeben hat. Der Zeitraum zwischen Auftreffen und Ablösung soll als Kontaktverweildauer verstanden werden. Eine längere Kontaktverweildauer begünstigend vermutlich die Ausbildung von unspezifischer Bindung und soll daher in einem magnetischen Beschichtungsvorgang minimiert werden. Noch unvorteilhafter ist der Effekt des Membrandurchdringens der Partikel als Ergebnis länger andauernder Einwirkung der Partikel auf die Zellmembran wird zum Beispiel in der Gentransfektion ausgenutzt (FOURIKI, A. [u.a.]: Evaluation of the magnetic field requirements for nanomagnetic gene transfection. Nano Rev. (2010) 1, 1-5).

Für die Zellseparation ist allerdings dieser Effekt nachteilig, da bereits magnetisch abgeschiedene Zellen durch die Partikeleinwirkung zerstört werden können, wie in Beispiel 2 verdeutlicht. Förderlich ist daher vergleichsweise sanfte magnetische Krafteinwirkung während der magnetischen Beschichtung und durch die magnetische Immobilisationsvorrichtung. Des Weiteren ist die Frequenz der Kollision eines Partikels mit einer Zelle pro Zeiteinheit zu brachten. Eine hohe Frequenz mag von Vorteil für das Zustandekommen einer lokalen Bindungsmöglichkeit unter der Annahme der positiven Korrelation höherer Wahrscheinlichkeit auf Ausbildung spezifischer gegenüber der unspezifischen Bindung sein. Im System der magnetisch beeinflussten Partikelbeschichtung ist die lokale Kollisionsfrequenz minimiert, jedoch die globale Kollisionsfrequenz also der Kollisionsanzahl eines Partikels mit mehreren Zellen über einen bestimmten Zeitraum vergleichsweise erhöht. Ähnlich der lokalen Kollisionsfrequenz ist eine hohe globale Kollisionsfrequenz bindungsförderlich. Es ist anzunehmen, dass eine hohe Kollisionsanzahl mit möglichst vielen Reaktionspartnern die Bindungswahrscheinlichkeit weiter erhöht, da hier biologische Unterschiede, wie zum Rezeptordichte und physikalische Unterschiede, wie zum Beispiel Partikel Reaktivität überwunden werden können (der Begriff "Partikel Reaktivität" soll im folgenden als Abscheidungsvermögen von Zielzellen mittels einer geeigneter magnetischer Abscheidungsapparatur in Beziehung zu einer bestimmten Menge an Partikeln verstanden werden und soll damit durch die Anzahl der abgeschiedenen Zellen pro Mikroliter (µl) Partikelsuspension definiert werden). Diese Eigenschaft bedingt allerdings deutlich unterschiedliche Bewegung sprich Geschwindigkeiten zwischen biologischen Material und magnetischen Partikeln. Die erfindungsgemäße Inkubationsvorrichtung nimmt auf die benannten Eigenschaften des Kollisionsverhaltens der magnetischen Partikel durch die Regelbarkeit der Positionsveränderung relative zum magnetischen Feldgradienten sprich der Regelbarkeit des Abstandes zwischen Behälter und Magnet, die Möglichkeit der Drehung des Inkubationsbehälters genauer durch die Regelung und Veränderbarkeit der Drehgeschwindigkeit und die Art des magnetischen Aufbaus Einfluss. Die optimale Einstellung der Apparatur und des Bindungsverhaltens der Partikel richtet sich nach der magnetischen Suszeptiblität bzw. der Größe der magnetischen Partikel.

Unter den im vorherigen beschriebenen theoretischen Annahmen über beeinflussende Systemparameter, lassen sich die Vorgänge in den verschiedenen magnetischen Partikelsystemen genauer erklären. Das passive, sprich unbeeinflusste Inkubieren von Zellen mit magnetischen Partikeln ist momentaner Stand der Technik. Die Firma Miltenyi verwendet die kleinsten Partikel im Bereich von 50 bis 100 nm und schlägt magnetische Beschichtung in Ruhe und gekühlt vor. Die kleinen Partikel bilden eine sehr große virtuelle Oberfläche und ein sehr stabiles Kolloid in zellfreundlichen Pufferlösungen, dessen Bewegung sehr stark von der Brownschen Molekularbewegung beherrscht wird. Es ist anzunehmen, dass die Partikel eine hohe lokale Kollisionsfrequenz und geringe Kontaktverweildauer erzeugen. Es ist weiterhin anzunehmen, dass durch die hohe Partikelkonzentration und Art der Bewegung in Lösung die Ausbildung von spezifischer Bindung begünstigt und die magnetische Beschichtung beschleunigt wird. Relative große Partikel mit einem hydrodynamischen Durchmesser von 1 µm bis 4 µm werden von der Fa. Dynabeads zur magnetischen Beschichtung und Separation eingesetzt. Im Gegensatz zum Miltenyi-System, ist die Verwendung von großen Partikeln vorteilhaft für den nachfolgenden Aufwand der magnetischen Immobilisierungsvorrichtung. Der große Partikeldurchmesser wirkt sich allerdings nachteilig auf die Effizienz der magnetischen Inkubation aus. Die Partikel unterliegen sehr viel weniger Brownscher Molekularbewegung und müssen in regelmäßigen Abständen durch deren Sedimentation vermischt werden. Es ist anzunehmen, globale zu Ungunsten der lokalen Kollisionsfrequenz erhöht ist und die Kontaktverweildauer höher als bei kleinen Partikeln ist. Es ist anzunehmen, dass das Dynabead-System die Ausbildung von unspezifischer Bindung im Vergleich zu den kleinen Partikeln begünstigt. Dem kann im Normalfall mit dem Einsatz einer geringeren Anzahl an Partikeln entgegnet werden. Es wird jedoch trotzdem eine relativ hohe Partikelkonzentration benötigt, um ein Minimum an Kollisionswahrscheinlichkeit innerhalb üblicher Inkubationszeiten von 15 min bis 120 min zu garantieren, mit dem Effekt, dass die Partikelreaktivität absinkt. Fachin et al. beschreibt die Verwendung von Dynabead-Partikeln reaktiv gegen CD45, CD16 und CD66b zur Inkubation in Blutproben in Volumina von 7.5 ml bis 10 ml und der anschließenden Abscheidung der roten und weißen Blutzellen zwecks Anreicherung von seltenen Zellen mittels eines integrierten mikrofluidischen Chipvorrichtung (Fachin et a. 2017). Die roten Blutzellen wurden durch das sog. "deterministic lateral displacement"-Verfahren und die Weißen Blutzellen durch magnetische Trennung mittels negativer Selektion abgeschieden. Es wurde mindestens 5 ml Blut verwendet mit durchschnittlich 3x10⁷ depletierbaren kernhaltigen unerwünschten Zielzellen. Die magnetische Mindestladung zur erfolgreichen magnetischen Abscheidung wurde bei dieser Technologie mit 6 Partikeln beziffert. Es ergäbe sich aus dieser Mindestbeladung eine Anzahl von mindestens 1,8x10⁸ aufzuwendenden Partikeln, was unter Verwendung von Dynabeads einer Menge von 180 µg oder 18 µl für 5 ml Blut entspricht (Lieferkonzentration ist beispielsweise 7 - 12x10⁹ pro ml pro 10 mg, angenommen 1x10¹⁰ Partikel). In bestehenden Anwendungen wurde der Partikelaufwand mit 1.2 mg Dynabeads pro ml Blut angegeben, woraus sich eine Beschichtungseffizienz von 3% und signifikante Kosten bei höheren Probevolumina ergibt. Verbesserung mit Bezug auf Inkubationsdauer und Höhe der magnetischer Beschichtung sprich Partikelreaktivität wurden durch den permanenten und Einfluss eines magnetischen Feldgradienten und zentrischer Behälterrotation vorgeschlagen (Schreier et al. 2017). Dieses sogenannte magnetische dynamische Beschichtungsverfahren (abgekürzt DML) erklärt sich mittels obig genannter Theorie durch ein vergleichsweise verändertes Kollisionsverhalten für Partikel im 100 nm Bereich zu der Art der Inkubation in Ruhe. In diesem Verfahren ist anzunehmen, dass der Einfluss der Brownschen Molekularbewegung durch den magnetischen Einfluss auf die Partikel stark reduziert ist. Die Zufallsbewegung und lokale Kollisionsfrequenz ist zu Gunsten der globalen Kollisionsfrequenz und Kontaktdauer erniedrigt. Im Besonderen lässt sich durch die hohe globale Kollisionsfrequenz eine hohe Beschichtungseffizienz ableiten, aber ebenso die wahrscheinliche Ausprägung von unspezifischer Bindung durch erhöhte Kontaktverweildauer. In einer Validierungsstudie zur negativen Selektion unter Verwendung von 100 nm normalgroßen Partikeln wurde gezeigt, dass 3log Abreicherung von Leukozyten durch wiederholtes Inkubieren innerhalb von 35 Minuten moeglich ist. Allerdings war das Verfahren auf die Maximierung der Abreicherungseffizienz und Partikelreaktivitaet bestrebt. Es ergab sich eine Partikeleffzienz von 3x10⁵ Zellen pro µl Partikellösung bemessen (Partikelkonzentration, ca. 1,4x10⁹ Partikel pro µl). Es wird angenommen, dass für das bestehende System eine Partikelbeschichtung von 1000 Partikeln ausreicht. Daraus würde sich eine Partikel-Beschichtungseffizienz von 21.4% ergeben. Allerdings und erwartungsgemäß wurden Verluste von gewünschten Zellen bei sehr hoher Partikelreaktivität eingeräumt. Mit der Weiterentwicklung und dem Versuch die Vorgänge im dem DML-Prozess zu verstehen, haben sich mittels geeigneter Apparatur entscheidende Verbesserungen erreichen lassen. Entscheidend hierbei ist die Annahme und Erkenntnis der Fähigkeit der Verhinderung von unspezifischer Bindung durch optimale Einwirkung eines magnetischen Feldes auf den magnetischen Inkubationsvorgang. Dieser Vorgang ist Gegenstand der Erfindung mit geeigneter Apparatur. Die Erfindung bietet somit einen Lösungsansatz zu dem Anreicherungsdilemma und zielt auf die Erhöhung der magnetischen Beschichtungseffizienz und gleichzeitiger Verminderung der Ausprägung unspezifischer Bindung ab. Die Erzeugung hoher Kollisionsfrequenzen eines Partikels mit mehreren Bindungspartnern über den Zeitraum eines Inkubationszyklus steht dabei im Mittelpunkt zur Erhöhung der Beschichtungseffizienz. Es wird vermutet, dass im Gegensatz zu einer hohen Kollisionsfrequenz die Wahrscheinlichkeit auf spezifische Bindung innerhalb eines gegebenen Zeitraums durch die Kollision mit in der Zellsuspension befindlichen Bindungspartnern überragend höher ist. Gleichzeitig muss aber dafür gesorgt werden, dass der Grad der unspezifischen Bindung durch die Art und Anwendung des magnetischen Feldes herabgesenkt wird. Es wird vermutet, dass die Kontaktdauer und damit die Druck und Zugkräfte entscheidende Parameter zur Beeinflussung der unspezifischen und der spezifischen Bindung sind.

Im Folgenden sind die wesentlichen Merkmale der Erfindung ausgeführt, die zu den Verbesserungen gegenüber dem Stand der Technik, wie eine Lösung des Anreicherungsdilemmas, Zeit und Kostenaufwand, technische Automatisierung und Einfachheit des Assays geführt haben. Basierend auf der Annahme des unterschiedlichen Bindungsverhaltens zwischen irreversibler unspezifischer und spezifischer Bindung in Abhängigkeit des Kollisionsverhaltens und den Protokollansprüchen zur Anreicherung von seltenen Zellen wurde eine Inkubationsapparatur basierend auf dem DML Verfahren entwickelt, um entsprechenden förderlichen Einfluss auf das Kollisionsverhalten zu erhalten. Ein dazu geeigneter Aufbau ist in den Figuren 1 bis 5 angegeben. Es ergab sich eine Apparatur die ein veränderbares magnetischen Feld bzw. an die Inkubationsvolumina anpassungsfähiges magnetischen Feld bereitstellt. Des Weiteren wurde die Idee der zentrischen Drehung von bereits bestehender Technologie aufgegriffen, allerdings richtet sich die neue Funktionalität auf die Beherrschbarkeit der unspezifischen Bindung.

Der Magnet und dessen Einsatz spielt die grundlegendste Rolle in der Inkubationsapparatur. Die Art, Anordnung und Dimensionen des bzw. der Magneten sind entscheidend für den Beschichtungserfolg als auch für die Reduzierung der unspezifischen Bindung. Grundsätzlich ist das erfundene Verfahren auf die Reduzierung der Prozessvolumina zwecks einfacherer Handhabbarkeit, Automatisierbarkeit und Kostenreduzierung ausgerichtet, was sich somit auf die Geometrie des Magneten auswirkt. Entscheidend ist jedoch für den magnetischen Aufbau die geeignete und optimale Einflussnahme auf möglichst alle magnetischen Partikel im System. Die optimale Einflussnahme bezieht sich das magnetophoretische Verhalten der Partikel und bedarf eines hohen bzw. relativ gleichverteilten Feldgradienten in vertikaler Ebene. Dies ist besonders von Vorteil zur gleichmäßigen Beschichtung entlang einer Volumensäule des Inkubationsbehälters. Es ergeben sich daher zwei wesentliche Eigenschaften; die der Stärke des magnetischen Feldgradienten, zur ausreichenden Einflussnahme auf die kleinen magnetischen Partikel mit bestimmter magnetischer Suszeptiblität und die der räumlich umfassenden magnetischen Feldstärke, um die Partikel trotz deutlicher räumlicher Unterschiede möglichst gleichmäßig magnetisch ansprechen zu können. Es ist anzunehmen, dass bestmögliche magnetische Separation durch höchstmögliche Werte für Feldgradient als auch die Feldstärke erreicht wird. Der Magnetaufbau richtet sich somit nach den Inkubationsvolumenverhältnissen und der magnetischen Suszeptiblität der eingesetzten magnetischen Partikelart. Variierbarkeit der Arbeitsvolumina und Partikeldurchmesser werden daher durch die Position und Größenanpassung des Magneten unterstützt. Die Dimension der Magnethöhe (z-Richtung) richtet sich somit nach der Volumensäulenhöhe des Inkubationsbehälters (bzw. der darin befindlichen Inkubationslösung) und bedingt eine mehrfache Dimensionserhöhung. Es soll angenommen und erreicht werden, dass das Probenvolumen damit vom magnetischen Feld vollends bzw. ausreichend eingeschlossen wird. Es soll angenommen werden, dass die Magnettiefe (y-Richtung) die magnetische Feldstärke in gewissen Abstand zum Magneten beeinflusst. Die Dimensionen richten sich daher nach dem Volumendurchmesser der Probenflüssigkeit bzw. dem Durchmesser des Probenbehälters.

Im Weiteren ist es für die erfindungsgemäße Inkubationsvorrichtung entscheidend die Position zwischen Magneten und Probenbehälter während der magnetischen Inkubation und dem Waschvorgang variieren zu können. Generell ist dabei anzunehmen, dass eine optimale Magnetsituation die spezifische Bindungseffizienz erhöhen und die Ausbildung unspezifischer Bindung erniedrigen kann. In einer Ausführung wird dies durch die motorisierte Bewegung des Magneten in vertikaler in einer weiteren in horizontaler Richtung bewerkstelligt. In einer weiteren Ausführung wird die Positionsänderung durch die motorisierte Bewegung des Inkubationsbehälters bewerkstelligt. Die Bewegbarkeit in vertikaler Richtung hat zum Zweck unterschiedliche Volumina bestmöglich magnetisch ansprechen zu können. Die Behälterposition wird im Verhältnis zum Magneten verstanden und wird so eingestellt, dass sich die Probe im Zentrum des Magnetkörpers befindet und erzeugt eine bessere Gleichverteilung der magnetischen Zugrichtung. So soll angenommen werden, dass optimale magnetische Bedingungen zur magnetischen Inkubation mittig in der vertikalen als auch horizontalen Ebene des Magneten anzutreffen sind. Die motorisierte Bewegung in horizontaler Richtung regelt den Abstand zwischen Probenbehälter und Magnet. Generell ist dabei anzunehmen, dass die kinetische Energie und Kraft der geradlinigen Bewegung der Partikel durch derartige Apparatur beeinflusst werden können. Im Allgemeinen soll daher angenommen werden, dass ein geringer Abstand hohe magnetische Zugkräfte erzeugt und die Assoziation von magnetischen Partikeln und biologischen Material erschwert und sich damit begünstigend für die Ausbildung von spezifischer Bindung auswirkt. Es ist auch zu vernehmen, dass eine optimale Magnetsituation sowohl die spezifische Bindungseffizienz erhöhen als auch die unspezifischen Bindung erniedrigen kann. Eine weitere Optimierung stellt dabei die Funktionalität des Neigens des Magneten in vertikaler Ebene zur bestmöglichen Anpassung des magnetischen Feldes an den Behälter mit konischer Form dar (vergl. Fig. 3).

Des Weiteren beinhaltet die Inkubationsapparatur die Funktionalität der Behälterdrehung. Der allgemeine Bereich der Drehgeschwindigkeit ist abhängig von der magnetischen Suszeptiblität der Partikel und nimmt Einfluss auf die Bindungskinetik, Bewegungsrichtung der magnetischen Partikel relative zu den nicht-magnetischen Material und trägt damit zur Regelung der Höhe der magnetischen Beladung bei. Genauer wird angenommen, dass die Drehgeschwindigkeit Einfluss auf die magnetophoretische Bewegungsfähigkeit des während des Beschichtungsprozesses magnetisierten biologischen Materials nimmt. So zum Beispiel werden nach sehr kurzer Zeit der Inkubation, Zielantigen-reiche Zellen durch die Wahrscheinlichkeit der erhöhten magnetischen Beladung magnetisch abscheidbar. Durch den Flusswiederstand der Pufferlösung und die Kollision mit anderen Zellen wirkt sich die Behälterdrehung magnetophoretisch vermindernd aus. Im schnellen Drehbereich setzt daher verzögert und verringert Fraktionierung des magnetisierten biologischen Materials ein. Im mittleren Drehgeschwindigkeitsbereich setzt deutliche Fraktionierung zwischen magnetischem und nichtmagnetischem biologischem Material ein. Wie bereits theoretisiert, wirkt sich ein weiterer Aspekt der Drehung auf die Kontaktdauer zwischen biologischen Material und magnetischen Partikeln aus. Es muss gelten, dass die magnetischen Partikel geradlinig dem Magnetfeldgradienten folgen und die Zellen durch die Drehung eine nichtlineare Bewegungsrichtung relativ zu den magnetischen Partikeln annehmen. Es wird daher angenommen, dass die Drehgeschwindigkeit negativ mit der Kontaktdauer zwischen Zellen und magnetischen Partikeln korreliert. Es ist daher weiter anzunehmen, dass eine sehr schnelle Drehung unspezifische bzw. spezifische Bindung vermindert. Der Theorie folgend werden unterschiedliche Drehgeschwindigkeiten für die Partikelzugabe, dem Inkubation bzw. des Waschvorgangs und der magnetischen Abscheidung eingesetzt.

Trotz des Bestrebens, die magnetischen Partikel während der Inkubationsphase bzw. der magnetischen Beschichtungsphase in Lösung zu halten, um die Kollisions- und damit Bindungswahrscheinlichkeiten zu erhöhen, weist der Vorgang im Dasein von sehr hohen Zellmengen bereits nach wenigen Sekunden Abscheidung bzw. Fraktionierung auf. Es ist daher nötig diese Partikelfraktion während des Inkubationsprozesses durch Vermischung wieder in Lösung zu befördern. Ein Mischvorgang ist daher nötig und erst beendet, wenn vollkomme Gleichverteilung der Partikel ähnlich des Anfangszustandes wieder hergestellt wurde. Eine geeignete Mischmethode kann durch das sog. Vortexen oder durch Pipettieren bewerkstelligt werden. Die Mischung wird in einer Ausführung durch die automatisierte Betätigung eine Plastikspritze mit Nadel bewerkstelligt. Pipettieren und Dispensieren der Probenlösung in zwei oder mehr Wiederholungsschritten hat sich als effizienter Lösung im Vergleich zum Vortexen ergeben. Die Inkubation und Durchmischung stellt einen Inkubationszyklus dar.

Es ist förderlich im Zuge der Entwicklung gleichartiger Assays, den Einsatz von magnetischen Partikeln zwecks Verminderung von USB und Probenkosten zu optimieren. Der limitierende Faktor hierbei ist der Grad der Abreicherung bzw. die absolute Anzahl von Zielzellen. In bisherigen Beobachtungen wurde festgestellt, dass bei Erhöhung der Zielzellkonzentration, die Reaktionskinetik sprich Partikelbeschichtungseffizienz signifikant absinkt (Waseem, Shahid, Rachanee Udomsangpetch, and Sebastian C. Bhakdi. "Buffer-Optimized High Gradient Magnetic Separation." Journal of magnetics 21.1 (2016): 125-132.). Mit anderen Worten werden über den gleichen Zeitraum pro Zielzelle weniger Partikel gebunden. Die Verminderung scheint in etwa linear zu sein. In der vorliegenden Erfindung verhalten sich die Bindungseigenschaften unter der Vorrausetzung der Majorität der Zielzellpopulation gegenteilig, so dass höhere Zielzellzahlkonzentrationen die weitere Reduzierung der Partikelmenge ermöglichen. Es lässt sich somit die Partikelbeschichtungseffizienz maximieren. Die Beobachtung stellt einen weiteren Teil zur Lösung des Anreicherungsdilemmas dar. Die positive Korrelation zwischen Zielzellkonzentration und Beschichtungserfolg bei gleichbleibender Partikelmenge lässt sich durch die hohe Gesamtanzahl an Kollisionen pro Inkubationszyklus erklären. Das Inkubationsvolumen ist dabei eine wesentliche Stellschraube zur Optimierung der erforderlichen Bead-Menge. Es hat sich gezeigt, dass eine Verdreifachung der Partikelreaktivität bei dreifacher Reduzierung des Volumens zu erreichen ist. Neben der Erhöhung der Partikelreaktivität wirkt sich dieser Effekt ebenfalls reduzierend auf die Ausbildung von USB aus. Es ist anzunehmen, dass mit weniger Partikeln mehr spezifische Bindung erreicht werden kann, so dass weniger Partikel als bei bisherigen Verfahren erforderlich sind. Das Inkubationsvolumen ist dabei eine wesentliche Stellschraube zur Optimierung der erforderlichen Bead-Menge. Es hat sich gezeigt, dass Verdreifachung der Partikelreaktivität bei 3-fach Reduzierung des Volumens zu erreichen ist. Für die Anreicherung von seltenen Zellen mittels negativer Selektion ist eine Separationseffizienzsteigerung maßgeblich unter der Bedingung der Wiedergewinnung der gewünschten Zellen. Die Erfindung bietet somit eine Lösung zur hohen Abreicherung bei geringem Verlust von gewünschten Zellen (Beispiel 4).

Der erfundene Anreicherungsprozess bezieht Erkenntnisse des dynamisch magnetischen Beschichtens mit ein und ist in explizite Prozessschritte unterteilt. Grundsätzlich besteht der Vorgang aus der magnetischen Inkubation, dem Waschen und der magnetischen Abscheidung. Es soll aus dem Folgenden hervorgehen, dass die Funktionalität der erfundenen Apparatur mit Verweis auf dem Zusammenspiel von Inkubations- und Abscheidevorrichtung eine entscheidende Rolle in der Lösung des Anreicherungsdilemmas darstellt. Sämtliche Prozesse werden innerhalb und mittels der Apparatur abgewickelt. Die magnetische Inkubation besteht aus der Wiederholung mehrerer Inkubationszyklen, die ihrerseits in magnetische Drehung und Durchmischung unterteilt werden. Der magnetischen Inkubation ist das Ziel gesetzt ein Höchstmaß an spezifischer Bindung pro Inkubationszyklus zu erzeugen. Dies geschieht allerdings bei hoher Bindungskinetik nicht ohne ein Mindestmaß an USB und erzwingt die Waschung der Partikelfraktion. Das Waschen verläuft ähnlich dem der magnetischen Inkubation, jedoch unter Eintrag zusätzlicher Inkubationslösung und der Zielsetzung Bindungsaufbau zu verhindern bzw. Bindungen rückgängig zu machen. Die magnetische Abscheidung folgt nach dem Inkubations- bzw. Waschvorgangs und wird von der Abscheidungsvorrichtung gehandhabt. Hierbei wird die Probensuspension in einem geeigneten Behältnis in Ruhe für eine Dauer im wenigen Minutenbereich im Einfluss des höchsten Magnetfeldgradienten gebracht. Geeigneter Weise reichen 1.5 bis 2 Minuten. Die Besonderheit des erfundenen Anreicherungsverfahrens ist die hohe Zellrückgewinnungsrate und unterstützt dabei die Gangart der positiven als auch negativen Selektion in der Verhinderung in Zellverlust und Erzeugung hoher Reinheitsgrade von gewünschten Zellen.

Der Vorgang beginnt im Detail mit der Einstellung der Zielzellkonzentration, geeigneter Weise durch Erfassung der Zellzahl im System und mittels Zentrifugation und Resuspendierung in einem bestimmten Volumen. Während dieses Prozesses kann ein Blockierschritt erfolgen, indem die Zellsuspension mit üblichen Blockierreagenzien für eine kurze Zeit im wenigen Minutenbereich vor der Zentrifugation versetzt wird. Der Blockierschritt kann sinnvollerweise für Proben mit sehr hohem Schmutz bzw. Partikelgehalt eingesetzt werden. Es folgt die Zugabe der magnetischen Partikel. Die Apparatur wird so eingestellt, dass maximale Mischung erreicht und Bindung bzw. Fraktionierung über einen Zeitraum von wenigen Sekunden verhindert wird. Sämtliche Einstellungen sind in Tabelle 1 für jeden Prozessschritt noch ein Mal schematisiert. Es folgt die magnetische Inkubation mittels eines Inkubationszyklus. Die Zielsetzung ist die Maximierung von spezifischer Bindung und erlaubt nun Bindung demnach Fraktionierung im magnetischen Feld über einen länger Zeitraum, jedoch sinnvollerweise nicht länger als die komplette Abscheidung des magnetischen Materials. Die Absicht in diesem Schritt ist die Kollisionsfrequenz der Partikel mit möglichst vielen Zellen durch die Mobilisierung der magnetischen Partikel zu gewährleisten, jedoch bei geringer magnetischer Zugkraft. Der Abstand zwischen Behälter und Magnet wird daher so eingestellt, dass diejenigen magnetischen Partikel mit höchster magnetischer Suszeptiblität innerhalb weniger Sekunden nicht magnetisch abgeschieden werden können und somit eine Höchstmenge an magnetischen Partikeln nach der Bead-Zugabe zur Bindung mit dem Zielmaterial zur Verfügung steht. Der Abstand und Drehgeschwindigkeit richten sich im Detail nach der Anreicherungsart und dem Anreicherungsziel. So zum Beispiel wird in der positiven Selektion eine biologisch verträgliche magnetischen Beladung, jedoch eine maximale Beladung mit möglichst Gleichverteilung unter den Zielzellen in der negativen Selektion benötigt. Im ersten Fall eignet sich die Einstellung einer höheren Drehgeschwindigkeit mit geringerem Abstand und umgekehrt im Fall der negativen Selektion. Der Inkubationsphase folgt der Vermischungsschritt mit veränderter Zielsetzung. Es soll die ursprüngliche Gleichverteilungssituation der magnetischen Partikel bzw. der Rückgängigmachung der magnetischen Fraktionierung erreicht werden und bedingt die erneute Änderung der Drehgeschwindigkeit und des Abstandes. In diesem Schritt wird die Drehgeschwindigkeit maximiert und der Einfluss des Magneten durch hohen Abstand minimiert. Ein Inkubationszyklus erreicht noch keine nennenswerten Grad an magnetische Beschichtung und damit Abreicherungseffizienz. Um den magnetischen Beladung zu erhöhen, bietet sich daher die mehrfache Wiederholung eines Inkubationszyklus an. Das Inkubationsverfahren erzeugt einen hohen Beschichtungsdruck und damit leichte höhere USB als in Ruhe. Ein Großteil der USB ist reversibel und unspezifisch gebundenen Partikel können durch einen intensiven Waschprozess von den gewünschten Zellen getrennt werden. In einer geeigneten Ausführung kann diese Waschprozedur in Gegenwart eines magnetischen Feldes stattfinden. Es soll vorausgesetzt werden, dass das Waschen die bereits in dem Inkubationsprozess abgeschiedene Zellfraktion betrifft, in der sich unter anderem die gewünschten Zellen befinden. Es soll weiterhin vorausgesetzt werden, dass die eigentliche Probe für den letzten Schritt der magnetischen Abscheidung als Überstand der magnetischen Partikelfraktion entfernt und hierfür in einen neuen Behälter mit ähnlicher Volumenfassung transferiert wurde. Zum Waschen der abgeschiedenen Partikelfraktion wird eine Waschlösung bestehend aus gepufferter zellfreundlicher Lösung in einem Volumen ähnlich jedoch nicht mehr als das des Probenvolumens zugegeben und magnetisch für nicht länger als bei der Inkubation abgeschieden. IN einer geeigneten Ausführung soll hier ein Maximum an magnetischer Kraft erzeugt werden, um jegliche Art von Bindung zu verhindern bzw. reversible USB rückgängig zu machen. Es folgt für die Einstellung der Inkubationsapparatur, die Drehgeschwindigkeit im Vergleich zur magnetischen Beschichtung anzuheben und den Behälterabstand zum Magneten zu minimieren. Dies wird durch den geringsten Abstand und im Falle von konischen Behältern die entsprechende Neigung des Magneten erreicht. Ein Waschzyklus ist mit dem Transfer der Waschlösung samt ausgewaschenen Zellen und anderen biologischen Material und der Zugabe zur einstigen Probenflüssigkeit abgeschlossen. Das Waschverfahren erzeugt damit eine Probenvolumenerhöhung je nach Anzahl der Wiederholungen. In einer geeigneten Ausführung hat sich eine Anzahl von drei Waschzyklen als ausreichend herausgestellt. Es folgt die magnetische Separation der Probe in Ruhe. Die technische Funktionalität zur magnetischen Abscheidung bedient sich zwecks Kosten und Platzersparnis in dem Apparat des Magneten der Immobilisationsapparatur und der Pipettierapparatur. Es wird höchstmögliche magnetische Kraft für die Magnetseparation benötigt. Die Abscheidungsrate kann durch Dauer geregelt werden - je kürzer desto weniger suszeptible und desto weniger beschichtete Zellen verbleiben in Lösung. Bei sehr langen Abscheidungsdauern wie zum Beispiel 5 min besteht allerdings die Wahrscheinlichkeit auch irreversibel unspezifisch gebundene Partikel abzuscheiden. Die Abreicherung kann im optimalen Fall im einstelligen Minutenbereich abgeschlossen werden und erreicht bis zu 2log Stufen. In dem finalen Vorgang der magnetischen Abscheidung kann in einer Ausführung die Probe in ein leeres gesondertes Behältnis in direkter Nähe zum Magneten mittels Pipettierapparatur überführt werden. Der Abscheidungsprozess geschieht in Ruhe und ist nach wenigen Minuten vollendet. In einer weiteren Ausführung kann die Plastikspritze der Pipettierapparatur als ein solches leeres Behältnis dienen und in direkter Nähe zum Magneten gebracht werden. Es ergibt sich der Vorteil der Einfachheit in Handhabung. Die Plastikspritze bietet darüber hinaus im Vergleich zu gängigen Plastikbehältern eine zur Abscheidung geeignetere Geometrie, die als Säulenform einen gleichbleibenden Radius bietet. Das Probenvolumen wird somit unabhängig der Volumenhöhe vom magnetischen Feld gleichmäßig angesprochen und erlaubt Volumenanpassungen und verspricht verbesserte Abscheidung. In einer weiteren Ausführung kann die Spritze parallel mit dem Magneten sinnvollerweise bis zu 50 Grad in Neigung gehen (Fig. 4). Die synchrone Neigung von Spritze und Magnet im Bereich 40 bis 50 Grad erhöht die Abscheidungseffizienz (der Begriff "Abscheidungseffizienz" wird in Log-Stufen angegeben und beschreibt das Verhältnis von Zielzellen vor bzw. nach Abreicherung; die Abscheidungseffizienz wird von zahlreichen Systemparametern, wie der Zellbiologie mit Bezug auf Rezeptor- oder Ligandenzugänglichkeit und -häufigkeit, der Anzahl der Zelltransferschritte und der Effizienz der magnetischen Abscheidungsapparatur beeinflusst). Die Neigung kann zu Beginn oder auch zu Beendigung der magnetischen Separation stattfinden. Sinnvoll ist dabei die Neigung mit der Spritze dem Magneten aufliegend. Es wurde in Versuchsreihen beobachtet, dass bereits abgeschiedene Zellen zum Zeitpunkt der Entnahme des Überstandes wieder in Lösung gehen. Genauer geschieht dieser Widereintritt in Lösung bei Flüssigkeitsentnahme im Falle eines separaten Behälters oder dem Ablassen im Falle der Verwendung der Spritze entlang der Luft-Flüssigkeit Schnittstelle bzw. Flüssigkeitsoberfläche (Fig. 5). Es handelt sich bei den wieder eintretenden Zellen um sehr leicht magnetisierte Zellen, deren magnetische Beladung nicht ausreicht, um die Luft-Flüssigkeitsschnittstelle zu überwinden und die somit dem Flüssigkeitsrückgang während der Flüssigkeitsentnahme oder dem Ablassen folgen und/oder in Lösung gehen. Die Funktionalität der synchronen Neigung setzt damit sinnvollerweise spätestens zum Zeitpunkt des Ablassens bzw. Entleerens der Spritze ein. Die verbesserte Abreicherungseffekt lässt sich durch den in Neigung zusätzlichen Halt der Spritzenwand erklären. Weiter bedarf es einer Wölbung am unteren Rand des jeweiligen Behälters (hier die Spritze), um die lose Fraktion von magnetisierten Zellen zu stoppen (Fig. 5).

Es lässt sich aus der Art der der erfundenen Anreicherungsmethode erschließen, dass der Grad der magnetischen Beladung in Abhängigkeit der jeweiligen Zellseparationsanwendung bzw. der sehr unterschiedlichen Systemsituation kontrolliert werden kann. Stellschrauben hierfür sind die Anzahl der Wiederholungen der Inkubationszyklen und innerhalb eines Inkubationszyklus die magnetische Zugkraft, demnach der die Drehgeschwindigkeit des Inkubationsbehälters und der Abstand des Magneten. Förderlich für Kosten, die Ausprägung von USB und Assaydauer ist das Bewusstsein einer Mindesthöhe der magnetischen Beladung und ist dabei abhängig von der magnetischen Ansprechbarkeitsschwelle durch die Abreicherungsapparatur. Zwecks optimaler magnetischer Beladung in Abhängig von der Zellseparationsanwendung werden unterschiedliche magnetische Inkubationskinetiken nötig. Für die negative Selektion lässt sich generell feststellen eine möglichst hohe und gleichmäßige magnetischen Beladungsdichte für sehr viele Zielzellen zu erreichen. Das Partikel zu Zellverhältnis ist vergleichsweise niedrig und es besteht die Gefahr der magnetischen Unterbeladung. Es muss daher auf schnelle Bindungskinetiken geachtet werden. In einer geeigneten Ausführung kann hierfür im Inkubationszyklus eine niedrigere Drehgeschwindigkeit bei sehr großem Abstand gewählt werden. In einer weiteren Ausführung eignet es sich die Inkubationsdauer zu verringern und die Wiederholungen anzuheben. In der positiven Selektion von sehr seltenen Zellen herrscht ein Übermaß an spezifisch bindenden Partikeln, so dass die Inkubation auf geringe Bindungskinetiken zur Vermeidung magnetischer Überbeladung eingestellt werden muss. In einer Ausführung kann der Abstand zwischen Behälter und Magnet verringert dabei jedoch der Drehgeschwindigkeit erhöht und die Anzahl der Wiederholungen von Inkubationszyklen reduziert werden.

Die Erfindung wird im Folgenden anhand von Ausführungsbeispielen weiter erläutert.

### Beispiel 1: Vergleich unterschiedlicher Magnetisierungseinrichtungen

Es wurden für zwei unterschiedliche Magnetisierungsvorrichtungen der Abscheidungsgrad und die Verringerung unspezifischer Bindung untersucht, die bei der Inkubation einer Suspension superparamagnetischer Partikel im Magnetfeld der jeweiligen Magnetisierungseinrichtung erreicht werden.

### Material:

- Neodymiumdauermagnet Typ 1 (herkömmlich): Feldstärke ca. 0,35 mTesla, Säulentyp, Einzelsegment mit Durchmesser von 24 mm und Höhe von 10 mm, maximal 3 Einzelsegmente zusammengefügt zu einer Gesamthöhe h (vergl. Fig. 1) von 30 mm
- Neodymiumdauermagnet Typ 2: 0,52 Tesla, quaderförmig, in Duopol-Anordnung, 30 mm x 30 mm x 30 mm pro Magnet
- Waschpufferlösung: isoosomolare Phosphat-gepufferte Lösung
- Superparamagnetische Partikel FluidMag, Nenngröße 100 nm (Chemicell GmBH, Deutschland)

### Durchführung:

Die parallele Anordnung zweier quaderförmiger Magnete gleicher Dimensionen mit gegenüberliegender Polarisierung und mit genau definiertem Abstand d zueinander (vergl. Figur 2) ist ein geeignetes Magnetsystem zur Bereitstellung eines hohen magnetischen Feldgradienten. Für eine quaderförmige Geometrie von Neodymiummagneten, wie hier gewählt, ist eine besonders hohe magnetische Flussdichte anzunehmen. Diese sogenannte Duopol-Anordnung weißt im Bereich des Zwischenraums (Spalt 21 in Fig. 2) der beiden Magneten, hier als die Abscheidungszone bezeichnet einen um den Faktor 10 höheren Feldgradienten als ein Einzeldauermagnet auf. Diese Art der Anordnung stellt somit gegenüber der Verwendung eines herkömmlichen Säulentyp-Magneten ein verbessertes magnetisches Felde zur Handhabung von kleinen magnetischen Partikeln mit Durchmessern im Bereich von 100 nm bis 200 nm dar. Die Breite und Tiefe des Magneten nimmt dabei Einfluss auf die magnetische Feldsättigung im Inkubationsbehälter und kann daher in Abhängigkeit des Behälterdurchmessers variieren. Bei einer Ausführungsform entspricht die Anordnung eines quaderförmigen Magnetpaares mit den Dimensionen von jeweils 30 mm x 30 mm x 30 mm einem maximalen Prozessvolumen von 500 µl bzw. 10 mm Säulenvolumenhöhe. Der Abstand der Magnetpaare zueinander ("d" in Fig. 2) wurde durch die Dimensionen des Probenbehälters unter Berücksichtigung der durch zunehmenden Abstand zunehmend schwächeren Feldgradienten festgelegt. Das Verfahren ist für die Prozessierung in geringen Volumina ausgelegt, weswegen hier herkömmliche zylindrische 1,5ml-Mikrozentrifugenplastikbehälter (-10,8 mm Durchmesser, -42 mm Höhe) als Probenbehälter verwendet wurden. Bei der gegebenen magnetischen Suszeptibilität der magnetischen Partikel und einem Durchmesser des Probenbehälters von nicht mehr als 8 mm, wie her verwendet, hat sich ein Abstand von maximal 3 mm als vorteilhaft erwiesen.

Weniger magnetisch wirksam, jedoch verwendbar sind Anordnungen von Einzelmagneten verschiedener Geometrien, wie beispielsweise eines Magneten vom Säulentyp. Die Abscheidungszone befindet sich dabei entweder auf der Nordpolseite oder der Südpolseite des Magneten.

In Abscheidungsversuchen mit magnetischen Partikeln in herkömmlichen gepufferten Lösungen wurde die Abscheidungszeit in Abhängigkeit des jeweiligen magnetischen Aufbaus untersucht. Dabei wurden 100 µg Partikelmaterial in 500 µl Puffer suspendiert und in einem herkömmlichen (durchsichtigen) 1,5ml-Mikrozentrifugenplastikbehälter dem magnetischen Feld der jeweiligen Magnetanordnung ausgesetzt. Dazu wurde der Behälter in ca. 3 mm Abstand zum Magneten gebracht. Gemessen wurde dabei die Zeit bis zur sichtbaren Klärung der Flüssigkeit im Behälter durch die magnetische Abscheidung der Partikel. Bei den magnetischen Anordnungen handelt es sich um: (1) einen Magneten vom Säulentyp mit A) 10 mm Höhe und B) mit 30 mm Höhe, (2) eine Duopol-Anordnung eines quaderförmigen Magnetpaars mit einem Abstand (Spalt) wischen den beiden Magneten von C) 0 mm, D) 3 mm bzw. E) 10 mm.

### Ergebnisse:

Die Versuche zeigten eine beschleunigte Abscheidung der Partikel bei Verwendung der Duopol-Anordnung mit Abständen von 0 mm und 3 mm. Dies weist auf eine erhöhte magnetische Ansprechbarkeit der Partikel unter diesen Bedingungen hin und darauf, dass eine verbesserte Magnetophoretik eine bessere Kontrollierbarkeit des Inkubationsvorgangs, sprich der Bewegung der Partikel erlaubt. Die vergleichsweise besten Ergebnisse wurden dabei mit der Duopol-Anordnung bei 3 mm Abstand erzielt.

**Tabelle 2: Magnetische Ansprechbarkeit bei unterschiedlichen Magnetanordnungen**

| Magnetanordnung | Säulentyp - Höhe: | | Duopol - Magnetabstand: | | |
|---|---|---|---|---|---|
| | 10 mm | 30 mm | 0 mm | 3 mm | 10 mm |
| Abscheidungsdauer in Sekunden | 130 | 100 | 45 | 45 | 100 |

### Beispiel 2: Abscheidungseffizienz und Rückgewinnung von Zielzellen mit anti-CD45-reaktiven magnetischen Partikeln

### Material:

- Inkubationspufferlösung: isoosmolare Phosphat-gepufferte Lösung mit 3% fötalem Kälberserum
- Waschpufferlösung: isoosmolare Phosphat-gepufferte Lösung
- RBC-Lysepuffer: 154 mM NH₄Cl, 10 mM NaHCO₃, 2mM EDTA
- Neodymiumdauermagent Typ 1 (herkömmlich): Feldstärke ca. 0,35 Tesla, Einzelsegment mit Durchmesser von 24 mm und Höhe von 10 mm, 6 Einzelsegmente zusammengefügt zu einer Gesamthöhe h von 60 mm, siehe Figur 1
- Neodymiumdauermagnet Typ 2: 0,52 Tesla, quaderförmig in Duopol-Anordnung, 30 mm x 30 mm x 30 mm pro Magnet,
- Eppendorf 1,5 ml Plastikröhrchen als Inkubationsbehälter
- 1 ml Plastikspritze
- Superparamagnetische Partikel, reaktiv gegen CD45 durch Beschichtung mit anti-CD45-Antikörpern (SanoLibio Walpurgisstraße 4, 81677 München, Deutschland)
- Vollblut von gesunden Spendern gelagert max. 24 h in Natrium-Heparin-Vacutainers
- Anreicherungsvorrichtung (Prototyp: SanoLibio Walpurgisstraße 4, 81677 München, Deutschland)

### Durchführung:

Zur Aufreinigung von Leukozyten aus frischen Vollblut wurden zunächst die Erythrozyten mittels eines geeigneten Lysepuffers (RBC-Lysepuffer) in üblicher Weise lysiert und die Zellsuspension anschließend zentrifugiert. Nachfolgend wurden die verbliebenen Zellen (im Wesentlichen humane periphere Leukozyten) 5 Minuten lang bei Raumtemperatur in der Inkubationslösung inkubiert.

Die weiteren Prozessschritte wurden entweder manuell oder mit dem neuen Verfahren unter Verwendung der Anreicherungsapperatur durchgeführt. Für alle Proben wurden bei Raumtemperatur jeweils die anti-CD45-reaktiven magnetischen Partikel mit 5x10⁶ der im vorhergehenden Schritt gewonnenen Leukozyten in einem Probenbehälter vermischt. Dabei wurden für alle Testproben drei Wiederholungen durchgeführt, jeweils 10 µl der konzentrierten Partikelsuspension (enthaltend 3,25 µg anti-CD45-Antikörper auf den Partikeln) und ein Gesamtinkubationsvolumen von jeweils 60 µl verwendet. Zur Bestimmung der Abscheidungseffizienz wurde die nicht-abscheidbare Zellfraktion im Überstand der Probe in das Verhältnis zur anfangs eingetragenen Zellmenge gesetzt. Zellzahlen wurde mittels Lichtmikroskop und Hämozytometer (Neubauer) bestimmt. Erwartungsgemäß befinden sich die Zellen nach der Anreicherung entweder im Überstand oder in der magnetisch abgeschiedenen Fraktion und ihre Summe nach der Abscheidung sollte theoretisch der anfangs eingetragenen Zellmenge entsprechen. Zur Bestimmung der Gesamtzellrückgewinnung wurde zusätzlich die Anzahl der Zellen in der Partikelfraktion bestimmt. Die Gesamtzellrückgewinnung ergibt sich somit aus dem Verhältnis der Gesamtzellzahl nach bzw. vor der Abscheidungsprozedur. Eine reduzierte Gesamtzellrückgewinnung weist auf Verluste durch Zellzerstörung hin.
A) Zum Vergleich mit der herkömmlichen Art der magnetischen Beschichtung wurden Testproben 5 Minuten lang in Ruhe inkubiert. Anschließend wurde die Zellsuspensionen unter Zugabe von jeweils 500 µl Waschpufferlösung mittels Pipettieren 3x durchmischt und der Probenbehälter zur magnetischen Abscheidung unverzüglich für die Dauer von 2,5 Minuten mit der Magnetanordnung vom Typ 2 zusammengebracht. Dieses Protokoll nimmt max. 8 Minuten in Anspruch.
B) Zum Vergleich mit dem aus Schreier et al. (Journal of Translational Medicine 15(1):6, 2017) bekannten dynamischen magnetischen Beschichtungsverfahren wurden Testproben (drei Wiederholungen) 5 Minuten lang wie in Schreier et al. beschriebenen inkubiert. Dabei wurde die Zellsuspension in den Einfluss eines magnetischen Gradienten (Magnet vom Typ1) gebracht und unter ständiger konzentrischer Drehung bei 4 rpm 50 Sekunden lang inkubiert und anschließend 10 Sekunden lang durchmischt. Dieser Vorgang aus Inkubation und Durchmischung wurde 5-mal wiederholt, was zu einer Gesamtinkubationszweit von 5 Minuten führte. Anschließend wurde der Inkubationskontainer aus dem Einflussbereich des Magneten entfernt. Die Mengenverhältnisse von Zellen und magnetischen Partikeln, sowie die Art und Dauer der Wasch- und Separationsprozedur entsprachen denen der Testreihe A.
C) Bei dem neuen Verfahren wurden die Testproben unter Verwendung der Anreicherungsvorrichtung mit den magnetischen Partikeln im Einflussbereich des Magnetfeldes (Magnetanordnung vom Typ 2) nicht länger als 5 Sekunden lang miteinander vermischt. Zur Erzeugung eines hohen Magnetfeldgradienten wurde der Abstand zwischen Probenbehälter und Magnet auf 0 mm eingestellt. Der Probenbehälter wurde währenddessen mit 300 rpm gedreht. Dem Partikeleintrag folgte die magnetische Beschichtung. Dazu wurde die Zell-Partikelsuspension bei einem Abstand zwischen Probenbehälter und Magnet von 2,5 mm für insgesamt 3,5 Minuten inkubiert. Ein einzelner Inkubationszyklus bestand dabei aus einer 20 Sekunden langen Inkubation unter konzentrischer Drehung im magnetischen Feld bei 2,5 rpm inkubiert und einer anschließenden Durchmischung unter Entnahme des Probenbehälters aus dem Magnetfeld. Dieser Vorgang aus Inkubation und Durchmischung wurde 7-mal wiederholt. Nach Abschluss der magnetischen Beschichtung und Abscheidung wurde der Überstand entfernt und bis zur magnetischen Abscheidung in einem geeigneten Behältnis (Transitcontainer) zwischengelagert. Es folgte eine Waschprozedur der während der Inkubation entstandenen abgeschiedenen Zellfraktion durch Zugabe von 100 µl Waschpufferlösung in den Probenbehälter und die gründliche Vermischung des magnetischen Pellets durch 4-maliges Pipettieren/Dispensieren und 15 Sekunden langes Inkubieren zur kurzzeitigen magnetischen Abscheidung. Dabei wurde der Einfluss des magnetischen Gradienten auf den Probenbehälter verstärkt, indem der Abstand zwischen Magnet und Probenbehälter von 2,5 mm auf 0 mm verringert wurde. Die Drehgeschwindigkeit betrug während des Mischvorgangs 300 rpm und während der 15 Sekunden dauernden magnetischen Abscheidung 3,5 rpm. Der Überstand wurde nach Abschluss eines Waschzykluses entfernt und erneut in den Transitcontainer überführt. Dieser Waschvorgang wurde noch 2-mal wiederholt und dauerte 2 Minuten. Die gewaschene Zell-Partikelfraktion (positiv selektierte Zellfraktion) wurde zur weiteren Analyse in 1 ml zellverträglichem Puffer resuspendiert. Die Überstandsfraktion im Transitcontainer bestand nunmehr aus einem Gemisch von nicht bzw. schwer magnetisch abscheidbaren Zellen, dessen Auftrennung intensiver magnetischer Abscheidungsbehandlung bedarf. Im Weiteren wurde der zwischengelagerte Überstand bzw. die Probe jeweils in eine 1 ml-Plastikspritze aufgenommen, darin mit zellverträglicher Lösung (Gesamtvolumen 1 ml) vermischt und 2,5 Minuten lang magnetisch abgeschieden. Der magnetischen Abscheidung folgte das Ablassen der fraktionierten Lösung bei einer Neigung der Spritze um 45°. Der Gesamtprozess dauerte somit ca. 8 min.
D) Zum Testen des Einflusses der magnetischen Feldbedingungen auf die neue Methode C wurden die in C) beschriebenen Versuche unter Verwendung eines Magneten vom Typ1 (statt der Typ2-Magnetanordnung) durchgeführt.

### Ergebnisse:

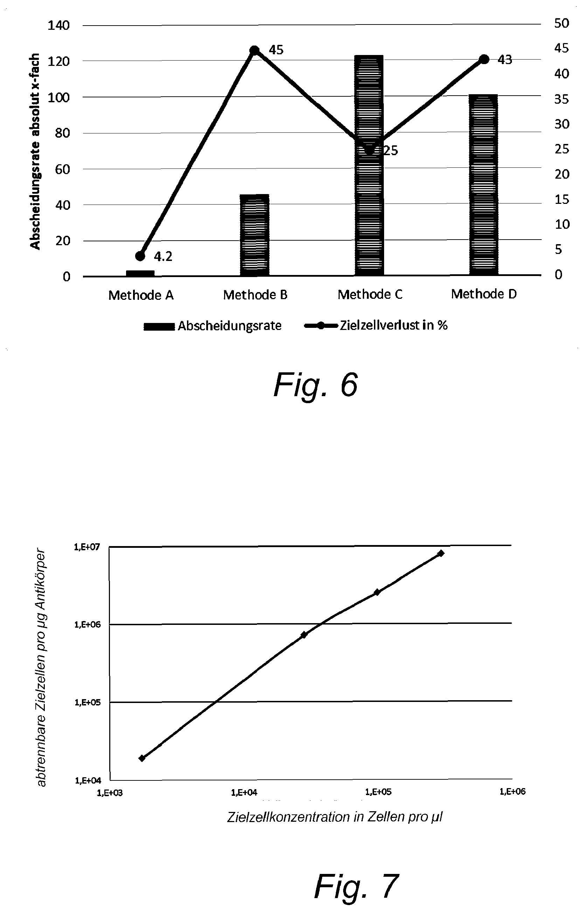

Mit diesem Versuch wurde die Abscheidungsrate und die Gesamtzellrückgewinnung bzw. der Verlust an Zielzellen in Abhängigkeit des Inkubationsverfahrens untersucht. Verluste von Zellen sind dabei auf die physikalische Einflussnahme der Partikel auf die Zellmembran während der Inkubationsphase bzw. der magnetischen Abscheidung begründet und korrelieren demnach mit der Zugkraft der magnetischen Partikel auf die Zellmembran. Diese Art von Zellverlust ist somit Indiz für die magnetische Beladungsdichte der Zellen bzw. die Zähigkeit der Zellmembran. Bei gegebener Menge an magnetischen Partikeln und Zielzellen lässt sich dadurch qualitativ auf die Höhe und Verteilung der magnetischen Beladungsdichte schließen. Figur 6 zeigt die Abscheidungsrate der Leukozyten aus der Suspension in Absolutwerten für die unterschiedlichen Inkubationsverfahren (Bestimmung mittels mikroskopischer Untersuchung, Olympus BX50). Des Weiteren wurde die Gesamtzellzahlrückgewinnung in % als Zielzellverlust aufgetragen.

Bei der Inkubation ohne Einfluss eines magnetischen Feldgradienten (Methode A) war der Zielzellverlust am geringsten und bei den Methoden B und D hingegen am höchsten. Die Methode C führte zu moderaten Zellverlusten. Es wird angenommen, dass der hohe Zielzellverlust bzw. die niedrige Gesamtzellrückgewinnung und die vergleichsweise geringere Abscheidung bei den Methoden B und D die Konsequenz hoher und ungleich verteilter magnetischer Beladungsdichte darstellen. Durch die Anwendung des neuen Verfahrens in Methode D erhöhte sich die Abreicherung im Vergleich zur Methode B deutlich. Die Verwendung eines im Vergleich zu Methode C niedrigeren Magnetfeldgradienten scheint zu einer deutlich höheren Beladungsdichte zu führen. Die Verringerung des Zellverlustes und die gleichzeitige vergleichsweise höhere Abreicherung bei der Methode C ist Indiz dafür, dass mit dieser Methode spezifische Bindung zu Ungunsten unspezifischer Bindung gefördert werden konnte. Generell lässt sich feststellen, dass im Gegensatz zur Inkubation in Ruhe, mit den Methoden B, C und D innerhalb sehr kurzer Zeit höchste Depletionswerte (40-fach bis 140-fach) erreicht werden konnten. Erwartungsgemäß ist die Beschichtung mit diesen Methoden wesentlich effizienter, sodass ein größerer Anteil an Zellen stärkeren magnetischen Kräften ausgesetzt ist. Die Methode C scheint in Anbetracht höchster Abscheidung und Beschichtungsgleichverteilung für die negativen Selektion von Zellen am geeignetsten zu sein.

### Beispiel 3: Untersuchung auf irreversible unspezifische Bindung magnetischer Partikel an Modeltumorzellen

### Material:

- wie in Beispiel 1
- zusätzlich:
   Modeltumorzellen: MDA-MB-231 (ATCC, HTB-26)
Die verwendeten magnetischen Partikel waren nicht mit einem Rezeptor beschichtet der eine spezifische Bindung mit der Zelloberfläche ermöglichen würde.

### Durchführung:

Es wurde eine Suspension magnetischer Partikel wie in Beispiel 1 beschrieben hergestellt, die jedoch zusätzlich 1x10⁴ Modelltumorzellen enthielt, und wie wie in Beispiel 1 beschrieben inkubiert. Es wurde davon ausgegangen, dass Modeltumorzellen, die sich nach einer magnetischen Abscheidungsdauer von 2,5 min in der abgeschiedenen Fraktion finden, irreversibel unspezifisch magnetisiert sind. Der Grad der unspezifischen Bindung wurde somit als Zellverlust durch Abscheidung ausgedrückt und versteht sich als die Menge an Zellen in der abgeschiedenen Fraktion samt den magnetischen Partikeln nach Abschluss der Anreicherungsprozedur. Des Weiteren wurde die Gesamtzellrückgewinnung wie in Beispiel 1 beschrieben untersucht. Dazu wurde die Anzahl der Zellen im Überstand nach der magnetsichen Abscheidung bestimmt. Zellzahlen wurden unter Verwendung eines Lichtmikroskopes und Haemozytometers (Neubauer) bestimmt.
A) Die hier verwendete Methode entspricht der in Beispiel 2 beschrieben Methode A.
B) Die hier verwendete Methode entspricht der in Beispiel 2 beschrieben Methode B.
C) Die hier verwendet Methode entspricht der in Beispiel 2 beschrieben Methode C.

### Ergebnisse:

Tabelle 3 zeigt den Zellverlust in %, welcher der Fraktion der (aufgrund unspezifischer Bindung) magnetisch abgeschiedenen Modeltumorzellen entspricht. Des Weiteren wurde die Gesamtzellrückgewinnung nach dem Verfahren bezogen auf die Ausgangszellmenge ermittelt. Die Gesamtzellrückgewinnung ergibt sich dabei aus der Summer der Zellzahlen im Überstand und der abgeschiedenen magnetischen Partikelfraktion. Eine Vergleichbarkeit an abgeschiedenen Modeltumorzellen ist nur bei einer sehr hohen Gesamtzellrückgewinnung (über 95%) gegeben. Die Ergebnisse weisen auf nur unbedeutende Zellverluste durch Zellzerstörung hin. Dies entspricht der Erwartung, da hier von einer geringen magnetische Beladungsdichte auszugehen ist. Bei diesem Experiment sind Tumorzellzahl, die Menge und Konzentration an magnetischen Partikeln und die Art und Dauer der magnetischen Abscheidung vergleichbar mit der möglichen Abscheidung von bis zu 99% spezifisch gebundenen Zellen. Die verwendete Partikelmenge ist extrem hoch und fördert die unspezifische Bindung. Es zeigt sich, dass das neue Verfahren (Methode C) zu wesentlich weniger unspezifischer Bindung als das bekannte Verfahren (Methode B) führt.

**Tabelle 3**

| **Methode** | **Zellverlust (Zellen in der abgeschiedenen Partikelfraktion)** | **Zellen im Überstand** |
|---|---|---|
| **A** | 5% | 98% |
| **B** | 36% | 64% |
| **C** | 8% | 94% |

### Beispiel 4: Partikelreaktivität in Abhängigkeit der Zielzellkonzentration

Das Experiment ist grundlegend zur Optimierung der zur Verfügung stehenden reaktiven Oberfläche an magnetischen Partikeln unter Berücksichtigung der gewünschten Verringerung an unspezifischer Bindung. Grundsätzlich tritt weniger unspezifische Bindung auf, umso geringer die Menge an magnetischen Partikeln ist. Es ist daher wünschenswert bei gleichbleibender Abscheidungseffizienz die Menge an magnetischen Partikeln verringern zu können.

### Material:

- wie in Beispiel 2

### Durchführung:

Das Experiment wurde wie die in Beispiel 2 beschriebene Methode C durchgeführt mit den folgenden Änderungen: Die verwendete Partikelmenge wurde auf eine Menge erhöht, die 3,75 µg auf dem Partikel gebundenem Antikörper entspricht, um größere Mengen an Zielzellen abzuscheiden. Das Inkubationsvolumen betrug 100 µl pro Probe. Die verwendete Menge an Zielzellen wurde im Bereich von 1.5x10⁵ bis 3x10⁷ Zellen pro Probe variiert, was einer Zielzellkonzentration von 1.5x10³ bis 3x10⁵ Leukozyten pro µl entspricht. Es wurde die Abreicherungseffizienz wie in Beispiel 2 beschrieben durch die Anzahl der verbliebenen bzw. nicht abgeschiedenen Leukozyten im Überstand nach der magnetischen Abscheidung bezogen auf die bekannten Menge der anfangs verwendeten Leukozyten bestimmt. Die Partikelreaktivität ergibt sich aus dem Verhältnis von abgeschiedener Menge an Leukozyten und verwendeter Menge an Partikeln bzw. Antikörpern (auf den Partikeln) und bestimmt die tatsächlich abscheidbare Menge an Zielzellen.

### Ergebnisse:

In vorhergehenden Experimenten wurde festgestellt, dass insbesondere bei der neuen Anreicherungsmethode, wie sie in Beispiel 2 als Methode C beschrieben ist, Änderungen des Inkubationsvolumens bei der magnetischen Beschichtung eine signifikante Auswirkung auf die Abscheidungseffizienz haben. Diese Eigenschaft kann man zur Reduzierung der Probenkosten und Verringerung der während des Anreichungsverfahrens verwendeten Volumina auszunutzen. Die genauere Untersuchung ergab einen linearen Zusammenhang zwischen der Zielzellkonzentration und der Partikelreaktivität, wie in Figur 7 verdeutlicht ist. Bei jeder der verwendeten Konzentrationen wurde eine Abscheidungsrate von 95% bis 97% erreicht. Durch Kenntnis des linearen Zusammenhangs zwischen Partikelmenge und Zielzellkonzentration ergibt sich die Möglichkeit die Abscheidungsrate in einer Genauigkeit von 1 bis 2% vorherbestimmen zu können bzw. die dafür nötige Partikelmenge berechnen zu können. Bei Änderung des Systems muss dazu jeweils ein Kalibrierungsexperiment in der Art, wie es in diesem Beispiel beschrieben ist, durchgeführt werden.

Figur 7 zeigt die Zielzellkonzentration als Funktion der Partikelreaktivität. Die höchste gemessene Partikelreaktivität wird bei der höchsten verwendeten Konzentration an Zielzellen erreicht. 1 µg Antikörper entspricht einer Partikelmenge, die zur Abreicherung von bis zu 8x10⁶ Zielzellen mittels der beschriebenen Methode ausreicht.

### Beispiel 5: Anreicherungseffizienz eines negativen Selektionsverfahrens

Die Anreicherungseffizienz drückt sich in der Wiedergewinnung und der (Sorten-)Reinheit der gewünschten Zellen in der angereicherten Probe im Vergleich zur Ausgangsprobe aus und wurde durch Spiking-Experimente ermittelt.

### Material:

- wie in Beispiel 2
- zusätzlich:
- zellverträgliche Pufferlösung (DMEM, phenolfrei, Gibco)
- Modeltumorzellen: MDA-MB-231 (ATCC, HTB-26)
- Anfärbungsreagenzien: CellTrace™ CFSE Cell Proliferation Kit, a-CD45PE (eBioscience), Hoechst Hoechst DNA stain (Merck Millipore)

### Durchführung:

Durch ein geeignetes Lyseverfahren und Zentrifugation wurden die roten Blutkörperchen entfernt und eine Suspension der verbliebenen Zellen (vor allem pheriphere Leukozyten) hergestellt und die Zellzahl in dieser Suspension mittels einer Zählkammer bestimmt (vgl. Beispiel 2). Zum eindeutigen Nachweis der Tumorzellen in einer Zellsuspension, die hauptsächlich aus Leukozyten besteht, wurden die Modelltumorzellen vor der Vermischung mit der Proben (d.h. der Leukozytensuspension) mit grünem CSFE-Fluoreszenzfarbstoff angefärbt. Speziell wurden zur Simulation einer Krebspatientenprobe 100 grün fluoreszierende Modelltumozellen mit 3x10⁷ (3-7,5 ml Vollblut) Leukozyten vermischt. Die absolute Reinheit der Modeltumorzellen betrug somit in der Theorie 0,00033%. Anschließend wurde ein Anreicherungsverfahren durchgeführt, wie es als Methode C in Beispiel 2 beschrieben ist. Dieser Vorgang wurde zweimal unter Modifikation des Inkubationsvolumens wiederholt. Beim ersten Anreicherungsschritt wurde (Beispiel 4 folgend) bestimmt, dass zum Erreichen einer Abreicherungsrate von 98,5% eine Menge von Partikeln zu verwenden ist, die 9,37µg Antikörper (auf den Partikeln) entspricht. Die Inkubation der Zellen mit den magnetischen Partikeln fand in einem Gesamtvolumen von 150 µl in Inkubationspuffer statt. Zur Messung des Tumorgehaltes vor der Anreicherung mittels des im Folgenden beschriebenen Verfahrens wurden der Probe (75 µl ohne magnetische Partikel) 2,5 µl entnommen und mit 35 µl zellverträglicher Pufferlösung verdünnt. Diese Probe (benannt als " Vor Anreicherung") beinhaltet in etwa die zu erwartenden 3 Modeltumorzellen und 1x10⁶ andersartige kernhaltige Zellen. Bei einer theoretischen Abreicherungsrate von 98.5% in der Probe "Nach Anreicherung" zählte der Leukozytenüberstand immer noch 4,5x10⁵ Zellen und die Reinheit der Modeltumorzellen betrug bestenfalls 0,022%. Beim zweiten und dritten Anreicherungsschritt wurde das Inkubationsvolumen mittels Pelletierung und Resuspendierung auf 50 µl verringert. Unter der Vorrausetzung eines Verhaltens des Partikelsystems wie es in Beispiel 4 beschrieben ist, ist die Partikelreaktivität durch die geringe Zielzellkonzentration und die Beschränkungen bei der Handhabung geringer Volumina relativ niedrig und beträgt in der Theorie maximal 1,5x10⁵ Zellen pro µg Antikörper (auf den Partikeln). Zur Abreicherung der verbleibenden Leukozyten wurde daher eine Partikelmenge verwendet, die 3,12ug Antikörper (auf den Partikeln) entspricht. Nach ca. 50 Minuten war der gesamte Anreicherungsprozess abgeschlossen und die Analyseproben "vor Anreicherung" sowie "nach Anreicherung" wurden mittels Zentrifugation auf ein Volumen von 30 µl eingestellt und anschließend mit Fluoresenzfarbstoffen zur Markierung von CD45 (Gelb) und Zellkern (Blau) angefärbt. Des Weiteren wurde eine Kontrollprobe hergestellt, um die tatsächliche Spiking-Konzentration festzustellen. Zur Probenanalyse wurde das Operatte Image Scanning System verwendet. Die Proben wurden dabei in eine 96-Well-Platte überführt und 10 min lang sedimentiert.

### Ergebnisse:

Es wurden für die Proben (positive Spiking-Kontrolle, Probe vor Anreicherung und Probe nach Anreicherung) Bilder der Gesamtfläche jeweils eines Wells in den Farbkanälen Gelb (für Leukozyten) und Grün (für die Modelltumorzellen) aufgenommen und einer manuellen Bildanalyse unterzogen. Die Rückgewinnung der Modeltumorzellen wurde anhand der positiven Kontrolle und der Probe "nach Anreicherung" ermittelt und ergibt sich aus dem Zellzahlverhältnis der Gesamtvolumina zwischen positiver Kontrolle und angereicherten Probe (nach Anreicherung). Für die Kontrollprobe und die angereicherte Probe wurde ein Gesamtvolumen von 70 µl eingestellt. Die Kontrollprobe beinhaltete 114 positive Ereignisse (d.h. grün fluoreszierende Modeltumorzellen) und die angereicherte Probe zählte 109 positive Ereignisse, was somit eine Zellrückgewinnung von 95,6% ergibt. Die Abreicherungseffizienz bezieht sich auf die magnetische Trennbarkeit der Zielzellen, hier den Leukozyten, und wird in log-Stufen angegeben. Die Abreicherungseffizienz ergibt sich aus dem Verhältnis der Anzahl kernhaltiger Zellen vor der Anreicherung und nach der Anreicherung. Zur Analyse wurde die Konzentration der Leukozyten in der Probe "nach Anreicherung" bestimmt. Die Extrapolation aus einer statistisch signifikanten Menge bei angenommener Gleichverteilung im Well ergab 2840 Leukozyten. Die Abreicherung betrug somit bei einer anfänglichen Zellzahl von ca. 3x10⁷ Leukozyten 4,02 log.

Die Anreicherungseffizienz gilt als ein aussagekräftiges Qualitätsmerkmal eines Anreicherungsverfahrens, da hier sowohl Zellwiedergewinnung als auch Abreicherung zu gleicher Zeit in Betracht gezogen werden. Die Anreicherungseffizienz wird somit durch das Verhältnis der Reinheitsgrade zwischen den Proben " vor und nach Anreicherung" beschrieben. Aus der Kontrollprobe und der Probe "vor Anreicherung" erschließt sich die anfängliche Zellzahl der beigemischten Modeltumorzellen. Beide Proben, die positive Kontrolle als auch die Probe vor Anreicherung sollten in etwa auf die gleiche Zellzahl kommen. Die Probe vor Anreicherung zählte 4 positive Ereignisse und ergab 120 Zellen auf das Gesamtvolumen extrapoliert. In der Probe vor Anreicherung waren 1.1x10⁶ kernhaltige Zellen enthalten. Die Reinheit der Modeltumorzellen vor bzw. nach Anreicherung betrug somit 0,00036% bzw. 3,84%. Zur Berechnung der Anreicherungseffizienz wurde daher die Reinheit der Probe nach Anreicherung durch die Reinheit der Probe "vor Anreicherung" geteilt und in log-Stufen transformiert. Es wurde eine Anreicherungseffizenz von 4,03 log ermittelt. Bei der Anreicherung von seltenen Zellen ist die absolute Reinheit gegenüber jeglicher Art überlappenden Analysesignals von großer Bedeutung. Bei der negativen Selektion mittels Leukozytenabreicherung verbleiben andere nicht-hämatopoietische Zellen in der angereicherten Probe und vermindern die Reinheit der gewünschten Zellen. Es wird im Allgemeinen davon ausgegangen, dass zur Durchführbarkeit molekularer Analysen eine Mindestreinheit von 1% notwendig ist. Beim vorliegenden Beispiel wurde eine zusätzliche Gesamtmenge von 710 CD45-negativen, kernhaltigen Ereignissen ermittelt und eine Gesamtreinheit von 3,07% ermittelt.

**Tabelle 4: Übersicht über die Anreicherungsqualität**

| **Parameter** | **Wert** |
|---|---|
| Zielzellzahl (Leukozyten) | 3x10⁷ Zellen |
| Tumorzellspike | 100 Zellen |
| Abreicherung in Zellzahl | 2840 Zellen |
| Abreicherung in log-Stufen | 4,02 log |
| Zell rückgewinnung | 95,6% |
| Reinheit gegen Leukozyten | 3,8% |
| Gesamtreinheit | 3% |
| Verfahrensdauer | 50 Minuten |
| Anreicherungseffizienz | 4,03 log |

### Beispiel 6: Analyse verbleibender Zellenpopulationen nach CD45-Depletion unter Verwendung der dynamischen magnetischen Beschichtungsmethode und dem negativen Selektionsprinzips

### Material:

- wie in Beispielen 2 und 5

### Durchführung:

Die Anreicherung erfolgte wie in den Beispielen 2 und 5 beschrieben. Es wurden für dieses Experiment 4,9 ml venöses Blut eines gesunden Spenders verwendet. Die angereicherte Zellen in der Probe wurde in 30 µl resuspendiert und in diesem Beispiel mit den folgenden Fluoreszenzfarbstoffen versetzt: Hoechst 33342 (Farbstoff zur Kenntlichmachung der Zellkerne, blauer Farbkanal), anti-CD45-PE-Konjugat (zur Identifizierung von Leukozyten, gelber Farbkanal), anti-CD71-FITC-Konjugat (zur Identifizierung von kernhaltigen Zellen der roten Blutzelllinie, grüner Farbkanal) und anti-Glycophorin-A-PerCPCy5-Konjugat (zur Bestätigung der Erythroblastenlinien, roter Farbkanal). Nach einer Inkubationszeit von 30 min bei 4°C zur farblichen Markierung wurde die Probe mit 1,5 ml zellverträglichem Puffer gewaschen, in 70 µl phenolfreiem DMEM resuspendiert und innerhalb von 0,5 h mittels Mikroskopie analysiert. Dazu würde die Probe in eine 96Well-Platte überführt und mittels des Perkin Elmer Operetta Imaging Systems analysiert. Belichtungszeiten und Lichtanregungsintensitäten wurden optimiert, um das sogenannte. Channel Bleeding zu minimieren.

### Ergebnisse:

In der angereicherten Probe lassen sich hauptsächlich vier wesentliche kernhaltigen Zellpopulationen feststellen. Die Häufigkeiten wurden durch manuelles Auszählen von 75% der aufgenommenen 355 Felder ermittelt, auf das Gesamtprobenvolumen von 4,9 ml extrapoliert und auf 1 ml normalisiert. Die Majoritätspopulation besteht mit ca. 127 Zellen pro ml aus Leukozyten, gefolgt von CD45-negativen sogenannten Pyrenozyten (McGrath et al., Blood, 2008, 111(4):2409-2417), einer zu den roten Blutzelllinien gehörigen Zelllart mit ca. 42 Zellen pro ml, klein- und großkernigen CD45-negativen Zellen mit ca. 19 Zellen pro ml und Erythroblasten mit 3,1 Zellen pro ml. Die Depletionsrate berechnet sich aus dem Verhältnis von Leukozyten vor und nach der Anreicherung und ergab 4,7log. Die Zellpopulationen nach Anreicherung sind in Tabelle 5 zusammengefasst.

**Tabelle 5: CD45-Depletionseffizienz**

| | |
|---|---|
| vor Anreicherung | 3,00 x 107 |
| nach Anreicherung | 620 |
| Depletionsfaktor | 4,84 x 10⁴ |
| log Depletion | 4,7 |
| | |

| verbleibende kernhaltige Körper: | |
|---|---|
| weiße Blutkörperchen | 66,0% |
| freie Nuklei | 22,2% |
| CD45-negative Zellen | 10,2% |
| Erythroblasten | 1,6% |

### Beispiel 7: Vergleich seltener Zellspektren nach CD45-Depletion zwischen Blutproben gesunder Individuen und Krebspatienten mittels Fluoreszenzmikroskopie unter Verwendung bekannter Zellmarker

### Material:

- wie in Beispiel 5

### Durchführung:

Die Anreicherung wurde wie in den Beispielen 2 und 5 beschreiben durchgeführt. Im nächsten Schritt nach der Anreicherung wurde die Probe auf ein Volumen von 30 µl eingeengt und in diesem Beispiel mit den folgenden Fluoreszenzfarbstoffen versetzt: Hoechst 33342 (Farbstoff zur Kenntlichmachung der Zellkerne, blauer Farbkanal), anti-CD45-PE-Konjugat (zur Identifizierung von Leukozyten, gelber Farbkanal), anti-CD326-(EpCAM)-Superbright-645 (zur Identifizierung von Epithelzellen, oranger Farbkanal), anti-CD71-FITC-Konjugat (zur Identifizierung von Erythroblasten, grüner Farbkanal) und anti-Glycophorin-A-PerCPCy5-Konjugat (zur Bestätigung der Erythroblastenlinien, roter Farbkanal). Die Analyse erfolgte wie in Beispiel 6 beschrieben.

### Ergebnisse:

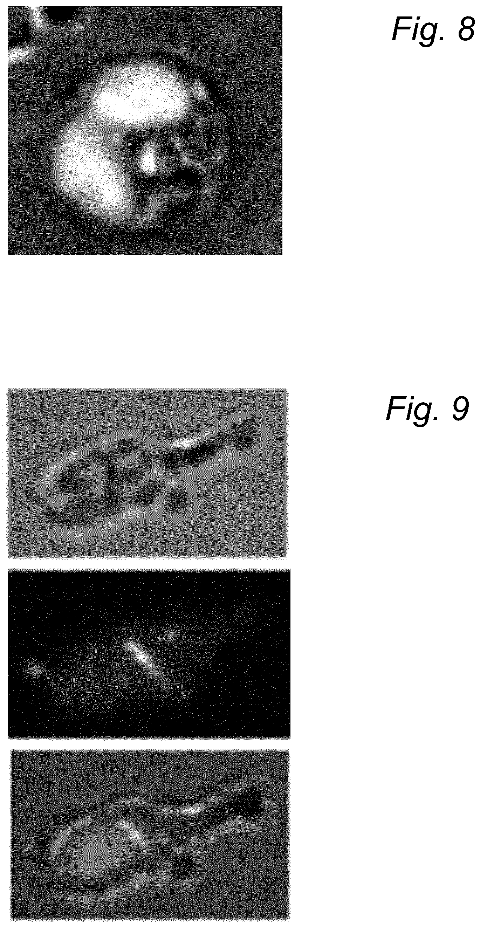

Allgemein wird durch Anreicherung die Detektion von seltenen Zellen mittels herkömmlicher Analyseverfahren ermöglicht. Die Anreicherungseffizienz korreliert dabei positiv mit der Nachweisbarkeit der Zellen von Interesse. Anreicherungsverfahren im 4- bis 5-log-Bereich ermöglichen bereits die Detektion von einzelnen Zellen in mehreren Milliliter Blut. Es wird angenommen, dass der hier beschriebene analytische bzw. diagnostische Vorgang zur Analyse seltener zirkulierender Zellen mit der Analyse eines pathologischen Gewebsschnittes verglichen werden kann. Dazu wird der angefärbte Gewebsschnitt auf bestimmte Zelltypen oder Zellmerkmale hin analysiert. Oft wird eine Parametergruppe von über 10 Parametern zur finalen Diagnose verwendet. Das hier verwendete Anreicherungsverfahren basiert auf der negativen Selektion und erlaubt die Kenntlichmachung einer beinahe vollständigen Auswahl der vorhandenen seltenen Zellen. Damit soll hiermit das Spektrum seltener Zellen als virtueller Schnitt durch das Blut betrachtet werden, der gutartige und bösartigen seltene Zellen umfasst. In diesem Beispiel wurde eine Auswahl von Antikörpern zur Kenntlichmachung der Zellen zusammengestellt, mit denen zum Beispiel der Zustand des Tumors und auch des Knochenmarks in Krebspatienten analysiert werden kann. Dabei wird zwischen gesunden Individuen und Krebspatienten anhand des Auffindens von Zellabnormität unterschieden. Für den Begriff Abnormität kann allerdings zum momentanen Stand des technischen Fortschritts bei der Analyse von seltenen Zellen keine absolut klare Trennung zwischen gesunden und kranken Menschen gefunden werden. Die hier verwendeten Kriterien schließen das Vorkommen von zirkulierenden Epithelzellen, Erythroblasten und anderweitig auffälligen CD45-negative Zellen mit ein. Im Allgemeinen sind im gesunden Organismus mitotische nicht-hämatopoietische Zellen (mit Nukleusteilung bzw. Zellmembranteilung) nicht anzutreffen, und sollen hier als aktive Zellen beschrieben werden, die auf Abnormität hindeuten. Figur 8 zeigt eine große sich teilende Zelle aus einem Magenkrebspatienten. Ebenfalls atypisch sind zirkulierende Epithelzellen in gesunden Individuen, deren Vorkommen im Krebspatienten dem Tumor zugeschrieben wird (Figur 9). Weniger eindeutig ist das Vorkommen größerer (über 13 µm) nichthämatopoietischer Zellen. Im Folgenden sind die unterschiedlichen Spektren von zwei Krebspatienten und zwei gesunden Individuen aufgelistet. Die Krebspatienten wurden bereits einer neoadjuvanten Therapie unterzogen. Den Spektren zufolge ließen sich diese Personengruppen deutlich voneinander unterscheiden. Insbesondere ließen sich trotz vorheriger Behandlung geringe Mengen von EpCam-positiven Zellen in den Krebspatienten identifizieren. Darüber hinaus war insbesondere bei der Brustkrebspatientin die hohe Anzahl aktiver Erythroblasten auffällig.

**Tabelle 6: Anzahl bestimmter Zelltypen pro ml Blut**

| **Untersuchte Person** | **Epithelzellen** | **Erythroblasten, normal** | **CD45-negative, mitotische Zellen** |
|---|---|---|---|
| **Magenkrebspatient in fortgeschrittenem Stadium** | 2,4 | 15,41 | 0,60 |
| **Brustkrebspatientin, Hormon-positiv, in fortgeschrittenem Stadium** | 0,3 | 445 | 0,30 |
| **gesunde Person 1** | 0 | 0,46 | 0 |
| **gesunde Person 2** | 0 | 0,47 | 0 |

### Bezugszeichenliste

- 10: Beladungsvorrichtung
- 11: Probenbehälter
- 12: zylindrischer Mantel des Probenbehälters
- 13: konischer Boden des Probenbehälters
- 14: Längsachse des Probenbehälters
- 15: drehbare Halterung des Probenbehälters
- 16: Antriebseinrichtung
- 17: Permanentmagnet
- 18: Längsachse des Permanentmagneten
- h: Höhe des Permanentmagneten
- 19: erster Pol des Permanentmagneten
- 20: zweiter Pol des Permanentmagneten
- 21: Spalt des Permanentmagneten
- d: Spaltbreite (Abstand der Pole)
- 22: Spritze
- 23: Halterung der Spritze
- 24: Halterung für weiteren Probenbehälter
- 25: Halterung für Fluidgefäße
- 26: weiterer Probenbehälter
- 27: Fluidgefäße
- 28: Hauptkörper der Spritze 22
- 29: zylindrischer Mantel des Hauptkörpers
- 30: Auslass
- 31: bauchiger Übergangsbereich
- 32: Zellfraktion
- 33: Überstand

## Patentansprüche

1. Verfahren zur Isolierung von gewünschten Zellen aus einer Probe nichtmagnetischer biologischer Materialien, welche eine Suspension gewünschter Zellen und unerwünschter Zellen umfasst, umfassend die Schritte:
- Zugabe von magnetischen oder magnetisierbaren Partikeln zur der Probe unter Einwirkung eines starken Magnetfeldgradienten, wobei die Partikel Oberflächenanteile aufweisen, die mit Zielzellen eine spezifische Bindung eingehen können, wobei die Zielzellen entweder die gewünschten oder die unerwünschten Zellen umfassen;
- Inkubation der Probe unter Einwirkung eines mittleren Magnetfeldgradienten, der niedriger als der starke Magnetfeldgradient des Zugabeschritts ist;
- Waschen der mit den Partikeln beschichtete Zellfraktion mit einer Waschlösung zur Verringerung unspezifischer Bindung;
- Abscheiden der Zielzellen aus der Probe unter Einwirkung eines starken Magnetfeldgradienten, der höher als der mittlere Magnetfeldgradient des Inkubationsschritts ist.

2. Verfahren gemäß Anspruch 1, wobei man die Probe während des Zugabeschritts der magnetischen oder magnetisierbaren Partikel mit einer schnellen Drehgeschwindigkeit rotieren lässt.

3. Verfahren gemäß Anspruch 2, wobei der Inkubationsschritt aus mehreren Inkubationszyklen besteht und jeder Inkubationszyklus
- einen magnetischen Beschichtungsschritt unter Einwirkung des mittleren Magnetfeldgradienten bei einer langsamen Drehgeschwindigkeit der Probe, die niedriger als die schnelle Drehgeschwindigkeit des Zugabeschritts ist, zur Erzeugung einer mit den Partikeln beschichteten Zellfraktion,
und
- einen Mischungsschritt zur Durchmischung der mit den Partikeln beschichteten Zellfraktion in der Suspension
umfasst.

4. Verfahren gemäß Anspruch 3, wobei der Mischungsschritt unter Einwirkung eines geringen Magnetfeldgradienten, der niedriger als der mittlere Magnetfeldgradient des Beschichtungsschrittes ist, bei einer schnellen Drehgeschwindigkeit der Probe, die höher als die langsame Drehgeschwindigkeit des Beschichtungsschritts ist, durchgeführt wird.

5. Verfahren gemäß einem der Ansprüche 3 oder 4, wobei der Waschschritt aus einem oder mehreren Waschzyklen besteht und jeder Waschzyklus die Schritte umfasst:
- Entfernen eines Überstandes der Suspension;
- Resuspendieren der verbleibende mit den Partikeln beschichtete Zellfraktion in der Waschlösung bei einem geringen Magnetfeldgradienten, der niedriger als der mittlere Magnetfeldgradient des Beschichtungsschritts ist bei einer schnellen Drehgeschwindigkeit, die höher als langsame Drehgeschwindigkeit des Beschichtungsschritts ist;
- Fraktionieren der mit den Partikeln beschichteten Zellfraktion aus der Suspension bei einem starken Magnetfeldgradienten, der höher als der mittlere Magnetfeldgradient des Beschichtungsschritts ist bei einer mittleren Drehgeschwindigkeit, die niedriger als die schnelle Drehgeschwindigkeit des Resuspendierschritts ist.

6. Verfahren gemäß einem der Ansprüche 1 bis 5, wobei man bei dem Abscheideschritt die Zielzellen magnetisch fixiert und die restliche Suspension entfernt.

7. Verfahren gemäß Anspruch 5, wobei die Zielzellen die gewünschten Zellen enthalten.

8. Verfahren gemäß Anspruch 6, wobei die entfernte Suspension die gewünschten Zellen enthält.

9. Verfahren gemäß einem der Ansprüche 1 bis 7, wobei die Partikel magnetisierbare superparamagnetische Materialien enthalten.

10. Verfahren gemäß einem der Ansprüche 1 bis 8, wobei man die Probe vor der Zugabe der magnetischen oder magnetisierbaren Partikel in einer Pufferlösung inkubiert, die Makromoleküle zur Sättigung unspezifischer Bindungsstellen enthält.

11. Vorrichtung zur Beladung von biologischem Material mit magnetischen oder magnetisierbaren Partikeln, umfassend
wenigstens eine drehbare Halterung zur Aufnahme eines Probenbehälters, die durch einen mit der Halterung gekoppelten Antriebsmotor in eine Drehung mit veränderbarer Drehgeschwindigkeit versetzt werden kann;
eine Magnetisierungseinrichtung, die am Ort des Probenbehälters ein Magnetfeld mit veränderbarer Feldstärke und veränderbarem Feldgradient erzeugen kann, und
eine Steuerungseinrichtung zur Steuerung des Antriebsmotors und der Magnetisierungseinrichtung.

12. Vorrichtung gemäß Anspruch 10, wobei der Probenbehälter eine im wesentlichen zylindrische Geometrie mit einer Zylinderachse aufweist und die Magnetisierungseinrichtung einen Permanentmagneten umfasst, der ein Magnetfeld erzeugt, dessen Feldstärke parallel zu einer Längsachse des Magneten konstant ist und in Ebenen senkrecht zu dieser Längsachse einen Feldgradienten ausbildet, wobei der Abstand der Längsachse des Magneten zur Zylinderachse des in der Halterung befindlichen Probebehälters veränderbar ist.

13. Vorrichtung gemäß Anspruch 11, wobei die Neigung der Längsachse des Magneten bezüglich der Zylinderachse des in der Halterung befindlichen Probenbehälters veränderbar ist.

14. Vorrichtung gemäß einem der Ansprüche 10 bis 12, die außerdem Mittel zur Isolierung von gewünschten Zellen aus der Probe biologischer Materialien umfasst, wobei die Mittel zur Isolierung eine beweglich montierte, von der Steuerungseinrichtung gesteuerte Spritze umfassen, die einerseits zum Austausch von Fluiden mit dem Probenbehälter in Wirkverbindung gebracht werden kann, und andererseits zum Ablassen von Fluiden mit der Magnetisierungseinrichtung in Wirkverbindung gebracht werden kann.

15. Vorrichtung gemäß Anspruch 13, wobei die Spritze neigbar montiert ist.

16. Vorrichtung gemäß einem der Ansprüche 10 bis 14, wobei der Probenbehälter und die Spritze zur Aufnahme von Fluidvolumina im Bereich von 10 µl bis 100 ml ausgelegt sind.
